# EUROPEAN PATENT SPECIFICATION

(11) **EP 2 486 053 B1**
(45) Date of publication and mention of the grant of the patent: **18.01.2017**
(21) Application number: 09788359.9
(22) Date of filing: 06.10.2009
(51) Int. Cl.: C07K 16/10

(54) **RSV-SPECIFIC BINDING MOLECULE**
RSV-SPEZIFISCHE BINDUNGSMOLEKÜLE
MOLÉCULE DE LIAISON SPÉCIFIQUE À RSV

(43) Date of publication of application: 15.08.2012
(73) Proprietor: MEDIMMUNE LIMITED, Cambridge Cambridgeshire CB21 6GH (GB)
(72) Inventor: BEAUMONT, Tim, NL-1191 GM Ouderkerk a/d Amstel (NL); BAKKER, Adrianus Quirinus, NL-1624 GM Hoorn (NL); YASUDA, Etsuko, NL-1108 HJ Amsterdam (NL)
(74) Representative: Rankin, Douglas
(86) International application number: PCT/NL2009/050599
(87) International publication number: WO 2011/043643

(56) References cited:
- WO-A1-2009/114815
- WO-A2-2004/043989
- WO-A2-2005/079479
- WO-A2-2008/147196
- WO-A2-2009/030237

## Description

The invention relates to the fields of biology and medicine.

Respiratory Syncytial Virus (RSV) is a common cold virus belonging to the family of paramyxovirus. RSV is virulent, easily transmissible and the most common cause of lower respiratory tract disease in children of less than 2 years of age. Up to 98% of children attending day care will be infected in a single RSV season. Between 0.5% and 3.2% of children with RSV infection require hospitalization. Approximately 90,000 hospital admissions and 4500 deaths per year were reported in United States. Major risk factors for hospitalization due to RSV are premature birth, chronic lung disease, congenital heart disease, compromised immunity, and age younger than 6 weeks in otherwise healthy children. No effective treatment of RSV positive bronchiolitis beside supportive care in the form of adequate nutrition and oxygen therapy is available. Antiviral therapies such as Ribavirin have not been proven to be effective in RSV infection. One monoclonal antibody, palivizumab (also called Synagis), is registered for prophylaxis against RSV infection. Palivizumab is a genetically engineered (humanized) monoclonal antibody to the fusion protein (F protein) of RSV. The F protein of RSV is a viral membrane protein and responsible for fusion of the virion with a host cell after attachment. In addition, infection of neighboring cells through the formation of syncytia is promoted by the F protein and its function is thought to depend on the original oligomeric structure of the protein. However, palivizumab is not always effective. Therefore, there is a need in the art for alternative and/or supplementary antibodies and therapies against RSV.

It is an object of the invention to provide improved antibodies against RSV, or functional equivalents of such antibodies. It is a further object to provide supplementary antibodies against RSV, which, in combination with existing RSV-specific antibodies, provide a synergistic effect. It is a further object of the invention to provide human or humanized antibodies or functional equivalents against the RSV F protein which are directed against an epitope that is different from the epitopes that known RSV-specific antibodies are directed against.

Accordingly, the present invention provides an isolated, synthetic or recombinant antibody or a functional part thereof which is capable of specifically binding Respiratory Syncytial Virus F protein and which comprises:
- a heavy chain CDR1 sequence comprising the sequence KLSIH,
- a heavy chain CDR2 sequence comprising the sequence
   GYEGEVDEIFYAQKFQH,
- a heavy chain CDR3 sequence comprising the sequence
   LGVTVTEAGLGIDDY,
- a light chain CDR1 sequence comprising the sequence RASQIVSRNHLA,
- a light chain CDR2 sequence comprising the sequence GASSRAT, and/
- a light chain CDR3 sequence comprising the sequence LSSDSSI.

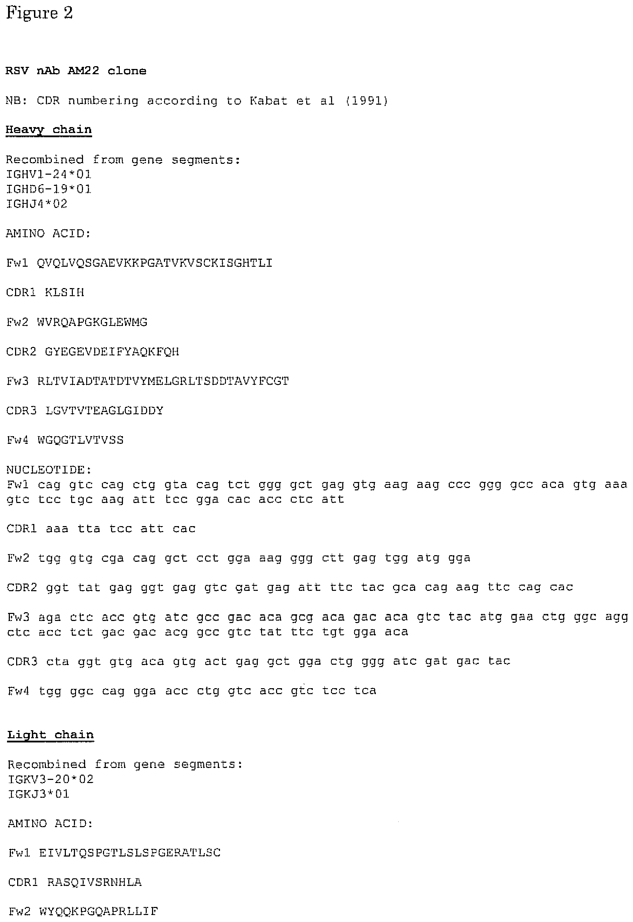

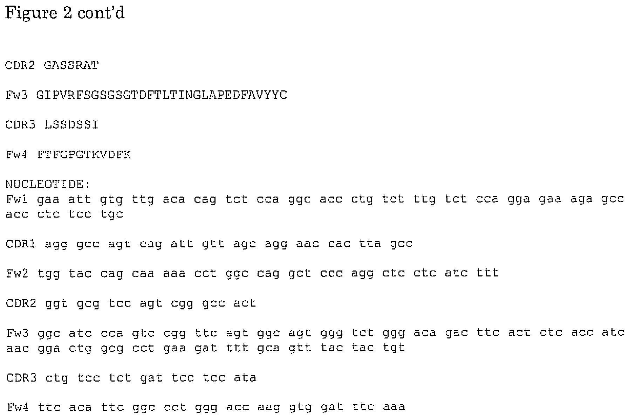

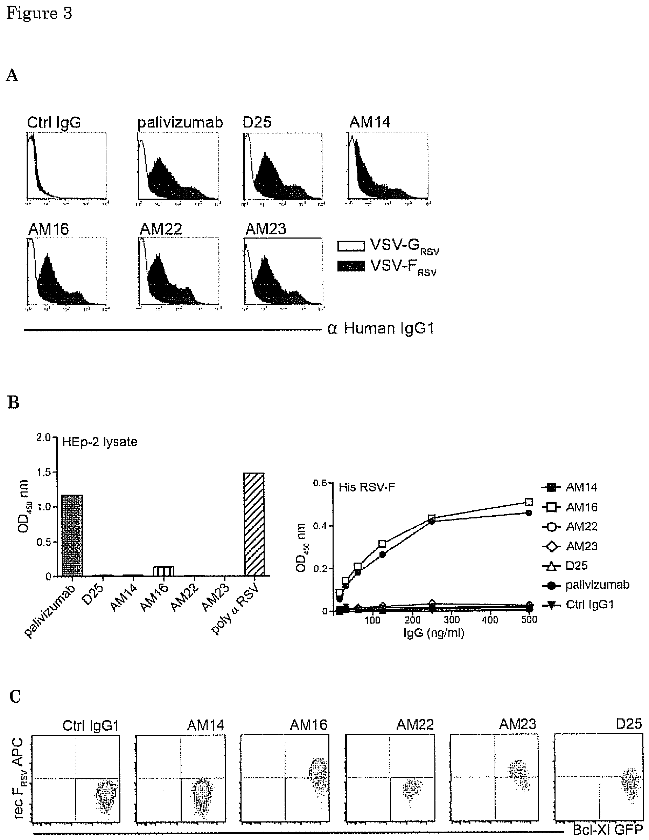

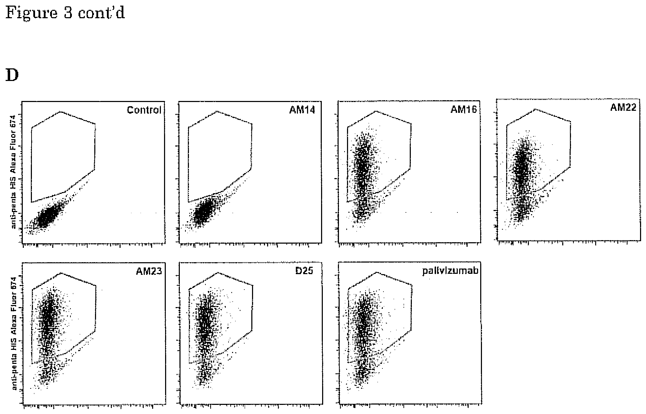

The present invention provides an antibody designated "AM22", which has heavy chain and light chain sequences as depicted in figure 2. The CDR sequences of AM22, which in particular contribute to the antigen-binding properties of AM22, are also depicted in figure 2. Antibody AM22 is fully human, is capable of specifically binding RSV (figure 3) and is therefore preferred for prophylactic and/or therapeutic use for human individuals.

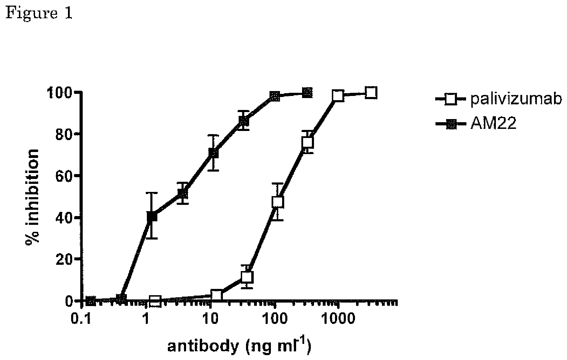

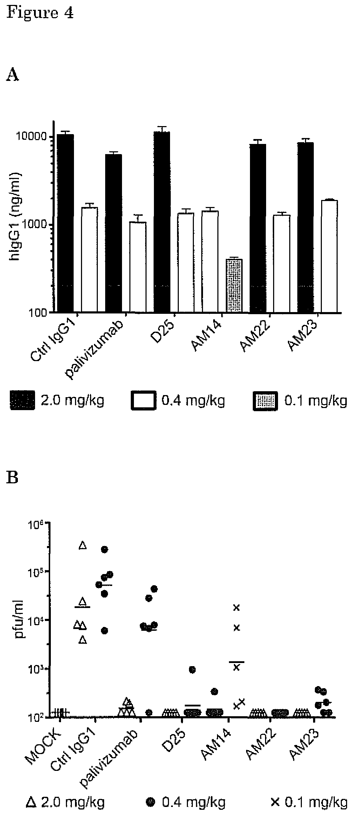

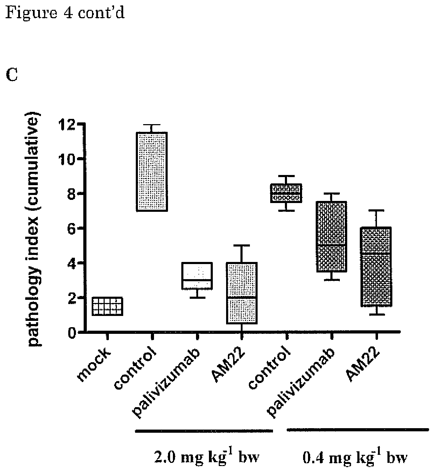

As mentioned above the only available clinically used anti-RSV antibody is palivizumab. This is a humanized monoclonal antibody directed against an epitope in the antigenic site of the F protein of RSV. Humanized antibodies still contain part of a mouse antibody and although immunogenic properties are diminished as compared to fully mouse antibodies, side-effects of humanized antibodies may occur when applied in humans. The present inventors, however, succeeded in obtaining and culturing human B-cells producing RSV-specific antibodies, so that human RSV-specific antibodies have been provided, that have strongly reduced - if at all - immunogenic activity as a result of the completely human sequence. As shown in the examples, antibodies according to the invention have superior characteristics as compared to palivizumab (figure 1 and table 1). The present inventors have shown that Cotton rats (*Sigmodon hispidus*) given antibodies according to the invention by intramuscular injection followed by intranasal challenge with RSV-X have a lower Pathology Index than Cotton rats given palivizumab followed by challenge with RSV-X (figure 4C and table 2). The Pathology Index used herein is a sum of scores to classify three individual markers for lung damage. These three markers are hyperthropy of bronchus and bronchioli epithelium, inflammation surrounding bronchus and bronchioli (peribronch(iol)itis) and inflammation in the alveoli (alveolitis). In addition, Cotton rats injected intramuscularly with antibodies according to the invention and subsequent RSV-X challenge had lower lung virus titers than Cotton rats given palivizumab followed by challenge with RSV-X (figure 4B), which was determined by TCID50 (50% tissue culture infective dose) assay. Hence, AM22 is preferred over palivizumab.

Besides palivizumab, some other RSV-specific antibodies are known. WO 2008/147196 discloses sequences of RSV binding molecules, namely antibodies D25, AM14, AM16 and AM23. As described in detail in example 1 of the current application, RSV specific antibody AM22 was obtained from the same donor as antibodies D25, AM14, AM16 and AM23. Strikingly however, AM22 recognizes RSV more efficiently than all other antibodies obtained from the same donor. The IC₅₀ value of AM22, 1.15 ng ml⁻¹, is lower than that of palivizumab, D25, AM14, AM16 or AM23. Therefore, the use of AM22 for treatment and/or prevention of a RSV-related disorder has advantages over the use of other RSV specific antibodies. Less AM22 antibody is necessary to obtain a similar effect compared to the other antibodies. Therefore, less AM22 has to be administered to an individual for treatment and/or prevention of a RSV-related disorder. Alternatively, with a similar amount of AM22, as compared to the other antibodies, a more effective treatment and/or prevention of a RSV-related disorder is achieved.

Furthermore, an RSV-specific antibody according to the present invention recognizes a different epitope as the previously disclosed RSV binding molecules. AM22, similar to the previously identified antibodies (WO2008/147196), is capable of binding the RSV F protein (figure 3A). However, AM22 does not bind a monomeric RSV F protein (figure 3B left and right panel). Contrary to AM22, the known antibodies palivizumab and AM16 (disclosed in WO 2008/147196 and figure 3B) are capable of binding the monomeric form of the F protein. Importantly the AM22 B cell line, expressing the antigen specific B Cell Receptor (BCR) does not recognize a recombinant form of the F protein (figure 3C). Thus AM22 binds a different epitope of the F protein than palivizumab, D25, AM23 and AM16. When the recombinant F protein was expressed in a vector containing an isoleucine zipper trimerization motif with eight HIS-tags (ILZ-8xHIS), then AM22 recognized this trimeric, conformation dependent structure (figure 3D). In contrast AM14 did not recognize either the monomeric form of the F protein or the ILZ-8xHIS F protein. Thus AM22 binds a different epitope of the F protein than AM14 as well. Furthermore it was found that AM22 did not interfere with D25 or palivizumab binding to RSV infected Hep2 cells. Thus, AM22 binds a different epitope of the F protein as compared to D25, AM14, AM16, AM23 and palivizumab. Therefore, RSV-specific antibodies or functional equivalents thereof according to the present invention are preferably combined with RSV-specific antibodies that are already known, such as palivizumab, D25, AM14, AM16 and AM23. By combining an antibody according to the invention with a known RSV-specific antibody, two or more different epitopes of RSV are recognized during the same therapy. This way, a stronger immunogenic response to RSV is obtained. Furthermore, higher antibody specificity against RSV is reached by combining one of the known RSV-specific antibodies with an AM22 antibody according to the invention. With a stronger immunogenic response to and higher specificity against RSV, such combination will result in more effective treatment and/or prevention of a RSV-related disorder. Finally, a lower overall antibody dosage is needed because AM22 has a stronger binding capacity for the F protein compared to palivizumab, D25, AM14, AM16 and AM23, as demonstrated by its low IC₅₀ value, of about 1.15 ng/ml.

This disclosure therefore provides an antibody or functional equivalent which has an IC₅₀ value less than 1.25 ng/ml in an *in vitro* neutralization assay wherein HEp-2 cells are infected with RSV-A2 virus. Said antibody or functional equivalent preferably has an IC₅₀ value of less than 1.2 ng/ml, preferably between 0.5 ng/ml and 1.2 ng/ml. Additionally, an antibody or functional equivalent according to the disclosure preferably has an IC₅₀ value which is at least 120-fold lower, more preferably at least 130-fold lower, than the IC₅₀ value of palivizumab in an *in vitro* neutralization assay wherein HEp-2 cells are infected with RSV-A2 virus. Said antibody or functional equivalent preferably has an IC₅₀ value of about 1.15 ng/ml. Thus with an RSV-specific antibody or functional equivalent thereof according to the present disclosure in combination with at least one available RSV-specific antibody a more effective treatment and/or prevention of a RSV-related disorder is achieved.

A functional equivalent of an antibody is defined herein as a functional part of an antibody.

A functional part of an antibody is defined as a part which has at least one same property as said antibody in kind, not necessarily in amount. Said functional part is capable of binding the same antigen as said antibody, albeit not necessarily to the same extent. A functional part of an antibody preferably comprises a single domain antibody, a single chain antibody, a single chain variable fragment (scFv), a Fab fragment or a F(ab')₂ fragment.

A functional derivative of an antibody is defined as an antibody which has been altered such that at least one property - preferably an antigen-binding property - of the resulting compound is essentially the same in kind, not necessarily in amount. A derivative is provided in many ways, for instance through conservative amino acid substitution, whereby an amino acid residue is substituted by another residue with generally similar properties (size, hydrophobicity, etc), such that the overall functioning is likely not to be seriously affected.

A person skilled in the art is well able to generate analogous compounds of an antibody. This is for instance done using a peptide library or phage display library. Such an analogue has essentially at least one same property as said antibody in kind, not necessarily in amount.

An antibody according to the invention is preferably a human antibody. The use of human antibodies for prophylaxis and therapy in humans diminishes the chance of side-effects due to an immunological reaction in a human individual against non-human sequences. In another embodiment an antibody, functional part, derivative or analogue according to the invention is a humanized antibody. Humanized antibodies are made by incorporating non-human hypervariable domains into human antibodies and therefore immunogenic properties are diminished as compared to fully non-human antibodies. In another preferred embodiment an antibody or functional part, derivative or analogue according to the invention is a chimeric antibody. This way, sequences of interest, such as for instance a binding site of interest, can be included into an antibody or functional equivalent according to the invention.

As is well known by the skilled person, a heavy chain of an antibody is the larger of the two types of chains making up an immunoglobulin molecule. A heavy chain comprises constant domains and a variable domain, which variable domain is involved in antigen binding. A light chain of an antibody is the smaller of the two types of chains making up an immunoglobulin molecule. A light chain comprises a constant domain and a variable domain. The variable domain is, together with the variable domain of the heavy chain, involved in antigen binding.

Complementary-determining regions (CDRs) are the hypervariable regions present in heavy chain variable domains and light chain variable domains. The CDRs of a heavy chain and the connected light chain of an antibody together form the antigen-binding site.

Now that the present invention provides the insight that the CDR sequences depicted in figure 2 provide desired binding characteristics, a skilled person is well capable of generating variants comprising at least one altered CDR sequence. For instance, conservative amino acid substitution is applied. It is also possible to alter at least one CDR sequence depicted in figure 2 in order to generate a variant antibody, or a functional equivalent thereof, with at least one altered property as compared to AM22. An antibody or functional equivalent may comprise a CDR sequence which is at least 70% identical to a CDR sequence as depicted in figure 2, so that the favorable binding characteristics of AM22 are at least in part maintained or even improved. A CDR sequence as depicted in figure 2 may be altered such that the resulting antibody or functional equivalent comprises at least one improved property, such as for instance an improved stability and/or binding affinity, as compared to AM22. The binding specificity is preferably maintained (in kind, not necessarily in amount). Variant antibodies or functional equivalents thereof may comprise an amino acid sequence which is at least 70% identical to a CDR sequence as depicted in figure 2. Various methods are available in the art for altering an amino acid sequence. For instance, a heavy chain or light chain sequence with a desired CDR sequence is artificially synthesized. Preferably, a nucleic acid sequence encoding a CDR sequence is mutated, for instance using random - or site-directed - mutagenesis.

Measurement of the affinity constant and specificity of binding between antigen and antibody is preferred in determining the efficacy of prophylactic, therapeutic, diagnostic and research methods using anti-RSV antibodies of the invention. "Binding affinity" generally refers to the strength of the sum total of the noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity can generally be represented by the equilibrium dissociation constant (Kd), which is calculated as the ratio k_{off}/kₒₙ. See, e.g., Chen, Y., et al., (1999) J. Mol Biol 293:865-881. Affinity can be measured by common methods known in the art, such as for instance a surface plasmon resonance (SPR) assay such as BiaCore or IBIS-iSPR instrument at IBIS Technologies BV (Hengelo, the Netherlands) or solution phase assays, such as Kinexa.

According to preferred embodiments, the present anti-RSV antibodies of the invention have binding affinities for an epitope on the RSV F protein that include a dissociation constant (K_{d}) of less than 1×10⁻²M, 1×10⁻³M, 1×10⁻⁴M, 1×10⁻⁵M, 1×10⁻⁶M, 1×10⁻⁷M, 1×10⁻⁸M, 1×10⁻⁹M, 1×10⁻¹⁰M, 1×10⁻¹¹M, 1×10⁻¹²M, 1×10⁻¹³M, 1×10⁻¹⁴M or less than 1×10⁻¹⁵M. In one embodiment, the anti-RSV antibodies have a K_{d} of less than 10⁻⁷M, less than 5x10⁻⁸M, less than 10⁻⁸M, less than 5x10⁻⁹M, less than 10⁻⁹M, less than 5x10⁻¹⁰M, less than 10⁻¹⁰M, less than 5x10⁻¹¹M, less than 10⁻¹¹M, less than 5x10⁻¹²M, less than 10⁻¹²M, less than 5x10⁻¹³M, less than 10⁻¹³M, less than 5x10⁻¹⁴M, less than 10⁻¹⁴M, less than 5x10⁻¹⁵M, or less than 10⁻¹⁵ M.

Disclosed herein is an isolated, synthetic or recombinant antibody or a functional part, derivative and/or analogue thereof, which comprises:
- a heavy chain CDR1 sequence comprising a sequence which has at least 70% sequence identity to the sequence KLSIH, and/or
- a heavy chain CDR2 sequence comprising a sequence which has at least 70% sequence identity to the sequence GYEGEVDEIFYAQKFQH, and/or
- a heavy chain CDR3 sequence comprising a sequence which has at least 70% sequence identity to the sequence LGVTVTEAGLGIDDY and/or
- a light chain CDR1 sequence comprising a sequence which is at least 70% identical to the sequence RASQIVSRNHLA, and/or
- a light chain CDR2 sequence comprising a sequence which is at least 70% identical to the sequence GASSRAT, and/or
- a light chain CDR3 sequence comprising a sequence which is at least 70% identical to the sequence LSSDSSI.

An antibody or functional equivalent may comprise a CDR sequence which is at least 75%, at least 80%, at least 85%, at least 90% identical to at least one of the CDR sequences depicted in figure 2. An antibody or functional equivalent according to the disclosure may comprise a CDR sequence which is at least 95% identical to at least one of the CDR sequences depicted in figure 2. The particularly preferred antibody AM22, described above, comprises CDR sequences which consist of the CDR sequences depicted in figure 2. An embodiment disclosed herein provides an isolated, synthetic or recombinant antibody or a functional equivalent thereof which is capable of specifically binding RSV and which comprises:
- a heavy chain CDR1 sequence comprising the sequence KLSIH, and/or
- a heavy chain CDR2 sequence comprising the sequence
   GYEGEVDEIFYAQKFQH, and/or
- a heavy chain CDR3 sequence comprising the sequence
   LGVTVTEAGLGIDDY, and/or
- a light chain CDR1 sequence comprising the sequence RASQIVSRNHLA, and/or
- a light chain CDR2 sequence comprising the sequence GASSRAT, and/or
- a light chain CDR3 sequence comprising the sequence LSSDSSI.

An antibody or functional equivalent may comprise the heavy chain CDR1 and CDR2 sequences and the light chain CDR1 and CDR2 sequences as depicted in figure 2, or sequences that are at least 70%, preferably at least 75%, more preferably at least 80%, more preferably at least 85% identical thereto. Further disclosed is therefore an isolated, synthetic or recombinant antibody or a functional part, derivative and/or analogue thereof which comprises a heavy chain CDR1 sequence comprising a sequence which is at least 70% identical to the sequence KLSIH and a heavy chain CDR2 sequence comprising a sequence which is at least 70% identical to the sequence GYEGEVDEIFYAQKFQH and a light chain CDR1 sequence comprising a sequence which is at least 70% identical to the sequence RASQIVSRNHLA and a light chain CDR2 sequence comprising a sequence which is at least 70% identical to the sequence GASSRAT. Said antibody or functional equivalent preferably comprises CDR sequences which are at least 75%, at least 80%, at least 85%, at least 90%, at least 95% identical to the above mentioned heavy chain CDR sequences and light chain CDR sequences. Said antibody or functional equivalent may also comprise a heavy chain CDR3 sequence comprising a sequence which is at least 70% identical to the sequence LGVTVTEAGLGIDDY, and/or a light chain CDR3 sequence comprising a sequence which is at least 70% identical to the sequence LSSDSSI. An antibody or functional equivalent comprising the above mentioned heavy chain CDR1, CDR2 and CDR3 sequences as well as the above mentioned light chain CDR1, CDR2 and CDR3 sequences is also disclosed.

Human CDR sequences may be optimized, in order to improve binding efficacy or stability. This is for instance done by mutagenesis experiments where after the stability and/or binding efficacy of the resulting compounds are preferably tested and an improved antibody or functional equivalent is selected.

Besides optimizing CDR sequences, it is often advantageous to optimize at least one sequence in at least one of the frame work regions. This is preferably done in order to improve binding efficacy or stability. Frame work sequences are for instance optimized by mutating a nucleic acid molecule encoding such frame work sequence where after the characteristics of the resulting antibody - or functional part - is preferably tested. This way, it is possible to obtain improved antibodies or functional parts. Isolated, synthetic or recombinant antibodies or functional parts, derivatives and/or analogues thereof comprising a heavy chain amino acid sequence which has at least 70% sequence identity to the heavy chain sequence as depicted in figure 2 are therefore also disclosed. Such heavy chain sequence provides desired binding properties, as evidenced by antibody AM22. Moreover, light chain amino acid sequences which have at least 70% sequence identity to the light chain sequence as depicted in figure 2 also provide desired binding properties, as evidenced by antibody AM22. Further disclosed is therefore an isolated, synthetic or recombinant antibody or a functional part, derivative and/or analogue thereof according to the disclosure having a heavy chain sequence comprising a sequence which has at least 70% sequence identity to the sequence QVQLVQSGAEVKKPGATVKVSCKISGHTLIKLSIHWVRQAPGKGLEWMGG YEGEVDEIFYAQKFQHRLTVIADTATDTVYMELGRLTSDDTAVYFCGTLGV TVTEAGLGIDDYWGQGTLVTVSS and/or having a light chain sequence which has at least 70% sequence identity to the sequence

EIVLTQSPGTLSLSPGERATLSCRASQIVSRNHLAWYQQKPGQAPRLLIFGA

SSRATGIPVRFSGSGSGTDFTLTINGLAPEDFAVYYCLSSDSSIFTFGPGTKV
DFK.

An isolated, synthetic or recombinant antibody or a functional part, derivative and/or analogue thereof according to the disclosure preferably comprises a heavy chain sequence and/or a light chain sequence which is at at least 90% identical to a heavy chain sequence and/or a light chain sequence as depicted in figure 2. The higher the homology, the more closely said antibody or functional equivalent resembles antibody AM22. An isolated, synthetic or recombinant antibody or a functional part, derivative and/or analogue thereof preferably comprises a heavy chain as well as a light chain which resemble the heavy and light chain of AM22. Further disclosed is therefore an isolated, synthetic or recombinant antibody or a functional part, derivative and/or analogue thereof comprising a heavy chain sequence and a light chain sequence which are at least 70%, more preferably at least 80%, more preferably at least 85%, more preferably at least 90%, most preferably at least 95% identical to the heavy chain sequence and the light chain sequence, respectively, as depicted in figure 2. In one embodiment an antibody or functional equivalent is provided which has a heavy chain sequence as depicted in figure 2 and a light chain sequence as depicted in figure 2.

One embodiment provides an isolated, synthetic or recombinant antibody or a functional part, derivative and/or analogue thereof comprising a heavy chain sequence consisting of the heavy chain sequence as depicted in figure 2, and/or comprising a light chain sequence consisting of the light chain sequence as depicted in figure 2. Alternatively, as is well known by the skilled person, it is possible to generate a shortened heavy chain or light chain sequence while maintaining a binding property of interest. Preferably, such a shortened heavy chain or light chain is generated which has a shorter constant region, as compared to the original heavy or light chain. The variable domain is preferably maintained. For instance, a Fab fragment or F(ab')₂ fragment or a single domain antibody or a single chain antibody or a nanobody or an unibody or a scFv fragment based on a heavy chain sequence or light chain sequence depicted in figure 2 is produced. A functional part of an antibody comprising at least a functional part of a sequence as depicted in figure 2 is therefore also provided. Said functional part has a length of at least 20 amino acids and comprises at least one sequence selected from the group consisting of a sequence which is at least 70% identical to the heavy chain CDR1 sequence depicted in figure 2 and a sequence which has at least 70% sequence identity to the heavy chain CDR2 sequence depicted in figure 2 and a sequence which has at least 70% sequence identity to the heavy chain CDR3 sequence depicted in figure 2 and a sequence which has at least 70% sequence identity to the light chain CDR1 sequence depicted in figure 2 and a sequence which has at least 70% sequence identity to the light chain CDR2 sequence depicted in figure 2 and a sequence which has at least 70% sequence identity to the light chain CDR3 sequence depicted in figure 2.

As said before, antibodies and functional equivalents according to the present invention recognize a unique epitope of an RSV F protein trimer. Hence, antibodies and functional equivalents are disclosed that specifically recognize this epitope. Antibodies or functional equivalents thereof that specifically recognize said unique epitope are preferably combined with RSV-specific antibodies that are already known, such as palivizumab, D25, AM14, AM16 and AM23. By combining an antibody or functional equivalent according to the invention that specifically recognizes said unique epitope with a known RSV-specific antibody, two or more different epitopes of RSV are recognized during the same therapy. This way, a stronger immunogenic response to RSV and/or a higher antibody specificity against RSV is reached With a stronger immunogenic response to and higher specificity against RSV, such combination will result in more effective treatment and/or prevention of a RSV-related disorder.

Therefore, disclosed herein is an isolated, synthetic or recombinant antibody or a functional part, derivative and/or analogue thereof which is capable of specifically binding an epitope that is recognized by an antibody which comprises:
- a heavy chain CDR1 sequence comprising the sequence KLSIH, and/or
- a heavy chain CDR2 sequence comprising the sequence
   GYEGEVDEIFYAQKFQH, and/or
- a heavy chain CDR3 sequence comprising the sequence
   LGVTVTEAGLGIDDY, and/or
- a light chain CDR1 sequence comprising the sequence RASQIVSRNHLA, and/or
- a light chain CDR2 sequence comprising the sequence GASSRAT, and/or
- a light chain CDR3 sequence comprising the sequence LSSDSSI.

Also disclosed herein is an isolated, synthetic or recombinant antibody or a functional part, derivative and/or analogue thereof which is capable of specifically binding an epitope that is recognized by an AM22 antibody which comprises:
- a heavy chain CDR1 sequence comprising the sequence KLSIH, and
- a heavy chain CDR2 sequence comprising the sequence
   GYEGEVDEIFYAQKFQH, and
- a heavy chain CDR3 sequence comprising the sequence
   LGVTVTEAGLGIDDY, and
- a light chain CDR1 sequence comprising the sequence RASQIVSRNHLA, and
- a light chain CDR2 sequence comprising the sequence GASSRAT, and
- a light chain CDR3 sequence comprising the sequence LSSDSSI.

Certain antibody constant region (Fc) modifications may alter effector functions. For example, the serum half-life of proteins comprising Fc regions is increased by increasing the binding affinity of the Fc region for FcRn. The term "antibody half-life" as used herein means a pharmacokinetic property of an antibody that is a measure of the mean survival time of antibody molecules following their administration. Antibody half-life can be expressed as the time required to eliminate 50 percent of a known quantity of immunoglobulin from the patient's body (or other mammal) or a specific compartment thereof, for example, as measured in serum, i.e., circulating half-life, or in other tissues. Half-life may vary from one immunoglobulin or class of immunoglobulin to another. In general, an increase in antibody half-life results in an increase in mean residence time (MRT) in circulation for the antibody administered. The increase in half-life allows for the reduction in amount of drug given to a patient as well as reducing the frequency of administration. To increase the serum half life of an antibody according to the invention, one may incorporate a salvage receptor binding epitope into the antibody (especially an antibody fragment) as described in U.S. Pat. No. 5,739,277, for example. As used herein, the term "salvage receptor binding epitope" refers to an epitope of the Fc region of an IgG molecule (e.g., IgG1, IgG2, IgG3, or IgG4) that is responsible for increasing the in vivo serum half-life of the IgG molecule. Alternatively, antibodies of the invention with increased half-lives may be generated by modifying amino acid residues identified as involved in the interaction between the Fc and an FcRn receptor (see, for examples, US Patent Nos. 6,821,505 and 7,083,784). In addition, the half-life of antibodies of the invention may be increased by conjugation to PEG or Albumin by techniques widely utilized in the art. In some embodiments antibodies comprising Fc variant regions of the invention have an increased half-life of about 5%, about 10%, about 15%, about 20%, about 25%, about 30%, about 35%, about 40%, about 45%, about 50%, about 60%, about 65%, about 70%, about 80%, about 85%, about 90%, about 95%, about 100%, about 125%, about 150% or more as compared to an antibody comprising a native Fc region. In some embodiments antibodies comprising Fc variant regions have an increased half-life of about 2 fold, about 3 fold, about 4 fold, about 5 fold, about 10 fold, about 20 fold, about 50 fold or more, or is between 2 fold and 10 fold, or between 5 fold and 25 fold, or between 15 fold and 50 fold, as compared to an antibody comprising a native Fc region. The disclosure therefore provides an antibody, or functional part, according the invention, comprising a salvage receptor binding epitope, and/or modified amino acid residues identified as involved in the interaction between the Fc and an FcRN receptor, and/or non naturally occurring amino acid residues. Another preferred embodiment provides an antibody or functional equivalent according to the invention which is conjugated to PEG or Albumin.

In one embodiment, the present disclosure provides Fc variants according to the invention, wherein the Fc region comprises a modification (e.g., amino acid substitution, amino acid insertion, amino acid deletion) at one or more positions selected from the group consisting of 234, 235, 236, 237, 238, 239, 240, 241, 243, 244, 245, 247, 251, 252, 254, 255, 256, 262, 263, 264, 265, 266, 267, 268, 269, 279, 280, 284, 292, 296, 297, 298, 299, 305, 313, 316, 325, 326, 327, 328, 329, 330, 331, 332, 333, 334, 339, 341, 343, 370, 373, 378, 392, 416, 419, 421, 440 and 443 as numbered by the EU index as set forth in Kabat et al (J Immunol. 1991; 147(5):1709-19). Optionally, the Fc region comprises a non naturally occurring amino acid residue at additional and/or alternative positions known to one skilled in the art (see, e.g., U.S. Patents 5,624,821; 6,277,375; 6,737,056; 7,083,784; 7,317,091; 7,217,797; 7,276,585; 7,355,008; 2002/0147311; 2004/0002587; 2005/0215768; 2007/0135620; 2007/0224188; 2008/0089892; WO 94/29351; and WO 99/58572).

In a specific embodiment, the present disclosure provides an Fc variant antibody according to the invention, wherein the Fc region comprises at least one non-naturally occurring amino acid at one or more positions selected from the group consisting of 252, 254, and 256. In one embodiment, the non-naturally occurring amino acids are selected from the group consisting of 252Y, 254T and 256E.

The present invention provides RSV-specific antibodies and functional equivalents thereof having improved properties as compared to prior art antibodies. The inventors have succeeded in generating RSV-specific antibodies with the lowest IC₅₀ value currently known. Such antibodies have a particular high or strong affinity for RSV and are therefore particularly suitable for counteracting and/or at least in part preventing an RSV-infection and/or adverse effects of an RSV infection. One embodiment therefore provides an antibody which has an IC₅₀ value of less than 1.25 ng/ml, preferably less than 1.2 ng/ml, more preferably less that 1.19 ng/ml, more preferably less than 1.18 ng/ml, and most preferably between 1.1 ng/ml and 1.17 as determined in the *in vitro* neutralization assay described in the examples (see figure 1).

Further disclosed herein is an isolated, synthetic or recombinant nucleic acid sequence or a functional equivalent thereof with a length of at least 15 nucleotides, preferably at least 30 nucleotides, more preferably at least 50 nucleotides, more preferably at least 75 nucleotides, encoding at least an antigen-binding part of an antibody or functional equivalent according to the invention. Such nucleic acid is for instance isolated from a B-cell which is capable of producing an antibody according to the invention. Disclosed is a nucleic acid sequence comprising a sequence which has at least 70% sequence identity to at least 15 nucleotides of a nucleic acid sequence as depicted in figure 2. A nucleic acid sequence comprise a sequence which has at least 75%, at least 80%, at least 85%, at least 90%, at least 95% sequence identity to at least 15 nucleotides of a nucleic acid sequence as depicted in figure 2. Said nucleic acid sequence as depicted in figure 2 may comprise at least one CDR encoding sequence.

Disclosed herein is an isolated, synthetic or recombinant nucleic acid sequence with a length of at least 15 nucleotides, or a functional equivalent thereof, encoding at least one CDR sequence of an antibody or functional equivalent according to the invention. Said nucleic acid sequence may encode at least one CDR sequence which has at least 70% sequence identity to a CDR region of antibody AM22. Nucleic acid sequences encoding AM22 CDR regions are depicted in figure 2. Further provided is therefore an isolated, synthetic or recombinant nucleic acid sequence, or a functional equivalent thereof, encoding the antibody or functional part thereof according to any one of the claims 1 to 4, 7 or 8 or which comprises the sequences aaattatccattcac, ggttatgagggtgaggtcgatgagattttctacgcacagaagttccagcac, ctaggtgtgacagtgactgaggctggactggggatcgatgactac, agggccagtcagattgttagcaggaaccacttagcc, ggtgcgtccagtcgggccact and ctgtcctctgattcctccata.

A nucleic acid sequence or functional equivalent may comprise a sequence which has at least 75%, at least 80%, at least 85%, at least 90% sequence identity to any of the above mentioned nucleic acid sequences. Further disclosed is a nucleic acid sequence or functional equivalent thereof comprising a sequence which has at least 70% sequence identity to at least part of a nucleotide sequence as depicted in figure 2, said part having at least 15 nucleotides and encoding at least one CDR region as depicted in figure 2.

A nucleic acid sequence or functional equivalent thereof may encode a region which has at least 70% sequence identity to an AM22 CDR region, an AM22 heavy chain and/or an AM22 light chain. Disclosed herein is an isolated, synthetic or recombinant nucleic acid sequence, or a functional equivalent thereof, comprising a sequence encoding an amino acid sequence which has at least 70% sequence identity to the sequence KLSIH, and/or at least 70% sequence identity to the sequence GYEGEVDEIFYAQKFQH, and/or at least 70% sequence identity to the sequence LGVTVTEAGLGIDDY, and/or at least 70% sequence identity to the sequence RASQIVSRNHLA, and/or at least 70% sequence identity to the sequence GASSRAT, and/or at least 70% sequence identity to the sequence LSSDSSI, and/or at least 70% sequence identity to the sequence
QVQLVQSGAEVKKPGATVKVSCKISGHTLIKLSIHWVRQAPGKGLEWMGG YEGEVDEIFYAQKFQHRLTVIADTATDTVYMELGRLTSDDTAVYFCGTLGV TVTEAGLGIDDYWGQGTLVTVSS, and/or at least 70% sequence identity to the sequence

EIVLTQSPGTLSLSPGERATLSCRASQIVSRNHLAWYQQKPGQAPRLLIFGA
SSRATGIPVRFSGSGSGTDFTLTINGLAPEDFAVYYCLSSDSSIFTFGPGTKV
DFK.

As said before, an antibody or functional equivalent according to the invention is capable of recognizing a unique epitope present on trimeric RSV F proteins. A nucleic acid sequence disclosed herein may encode a CDR sequence capable of specifically binding this unique epitope. Also disclosed is therefore an isolated, synthetic or recombinant nucleic acid sequence or a functional equivalent thereof, encoding at least one CDR sequence capable of specifically binding an epitope that is recognized by an antibody which comprises:
- a heavy chain CDR1 sequence comprising the sequence KLSIH, and/or
- a heavy chain CDR2 sequence comprising the sequence
   GYEGEVDEIFYAQKFQH, and/or
- a heavy chain CDR3 sequence comprising the sequence
   LGVTVTEAGLGIDDY, and/or
- a light chain CDR1 sequence comprising the sequence RASQIVSRNHLA, and/or
- a light chain CDR2 sequence comprising the sequence GASSRAT, and/or
- a light chain CDR3 sequence comprising the sequence LSSDSSI.

Said nucleic acid sequence may encode a whole antibody or functional equivalent (for instance comprising a heavy chain or light chain) according to the invention. Further disclosed is therefore an isolated, synthetic or recombinant nucleic acid sequence, or a functional equivalent thereof, encoding an antibody or functional equivalent thereof capable of specifically binding an epitope that is recognized by an antibody which comprises:
- a heavy chain CDR1 sequence comprising the sequence KLSIH, and/or
- a heavy chain CDR2 sequence comprising the sequence
   GYEGEVDEIFYAQKFQH, and/or
- a heavy chain CDR3 sequence comprising the sequence
   LGVTVTEAGLGIDDY, and/or
- a light chain CDR1 sequence comprising the sequence RASQIVSRNHLA, and/or
- a light chain CDR2 sequence comprising the sequence GASSRAT, and/or
- a light chain CDR3 sequence comprising the sequence LSSDSSI.

A nucleic acid sequence or functional equivalent may encode an antibody or a functional part, derivative and/or analogue thereof capable of specifically binding an epitope that is recognized by an AM22 antibody, which comprises a heavy chain CDR1 sequence comprising the sequence KLSIH, and a heavy chain CDR2 sequence comprising the sequence GYEGEVDEIFYAQKFQH, and a heavy chain CDR3 sequence comprising the sequence LGVTVTEAGLGIDDY, and a light chain CDR1 sequence comprising the sequence RASQIVSRNHLA, and a light chain CDR2 sequence comprising the sequence GASSRAT, and a light chain CDR3 sequence comprising the sequence LSSDSSI.

A nucleic acid sequence or functional equivalent may encode an antibody or functional equivalent thereof that has a dissociation constant (K_{d}) of less than 1×10⁻²M, 1×10⁻³M, 1×10⁻⁴M, 1×10⁻⁵M, 1×10⁻⁶M, 1×10⁻⁷M, 1×10⁻⁸M, 1×10⁻⁹M, 1×10⁻¹⁰M, 1×10⁻¹¹M, 1×10⁻¹²M, 1×10⁻¹³M, 1×10⁻¹⁴M or less than 1×10⁻¹⁵M.

Further provided is a vector comprising a nucleic acid sequence according to the invention. Such vector is suitable for a variety of applications. For instance, a vector of the invention comprising a therapeutically beneficial nucleic acid sequence is suitable for prophylactic or therapeutic applications. Administration of such vector to an individual in need thereof results in expression of said prophylactic or therapeutic nucleic acid sequence *in vivo.* Said vector can also be used in applications involving in *vitro* expression of a nucleic acid sequence of interest, for instance for (commercial) production of antibodies or functional equivalents according to the invention. Methods for constructing a vector with a nucleic acid sequence according to the invention are well known in the art. Non-limiting examples of vectors suitable for generating a vector of the invention are retroviral and lentiviral vectors.

The term "% sequence identity" is defined herein as the percentage of residues in a candidate amino acid sequence or candidate nucleic acid sequence that is identical with the residues in a reference sequence after aligning the two sequences and introducing gaps, if necessary, to achieve the maximum percent identity. Methods and computer programs for the alignment are well known in the art.

As used herein, a nucleic acid molecule or nucleic acid sequence of the invention preferably comprises a chain of nucleotides, more preferably DNA and/or RNA. In other embodiments a nucleic acid molecule or nucleic acid sequence of the invention comprises other kinds of nucleic acid structures such as for instance a DNA/RNA helix, peptide nucleic acid (PNA), locked nucleic acid (LNA) and/or a ribozyme. Such other nucleic acid structures are referred to as functional equivalents of a nucleic acid sequence. The term "functional equivalent of a nucleic acid sequence" also encompasses a chain comprising non-natural nucleotides, modified nucleotides and/or non-nucleotide building blocks which exhibit the same function as natural nucleotides.

A nucleic acid sequence or vector according to the present invention is particularly useful for generating antibodies or functional equivalents which are specific for RSV. This is for instance done by introducing such nucleic acid sequence or vector into a cell so that the cell's nucleic acid translation machinery will produce the encoded antibody or functional equivalent. In one embodiment, a nucleic acid sequence or vector encoding a heavy and/or light chain according to the invention is expressed in so called producer cells, such as for instance cells of a Chinese hamster ovary (CHO), NSO (a mouse myeloma) or 293(T) cell line, some of which are adapted to commercial antibody production. Proliferation of said producer cells results in a producer cell line capable of producing antibodies or functional equivalents thereof according to the present invention. Preferably, said producer cell line is suitable for producing antibodies for use in humans. Hence, said producer cell line is preferably free of pathogenic agents such as pathogenic microorganisms. Most preferably, antibodies or functional equivalents consisting of human sequences are generated using at least one nucleic acid sequence or vector according to the invention.

An isolated or recombinant antibody producing cell capable of producing an antibody or a functional part, derivative and/or analogue thereof according to the invention is therefore also provided, as well as a method for producing an isolated, synthetic or recombinant antibody or functional part, derivative and/or analogue according to the invention, comprising providing a cell with a nucleic acid sequence or functional equivalent or vector according to the invention and allowing said cell to translate said nucleic acid sequence or functional equivalent or vector, thereby producing said antibody or functional part, derivative and/or analogue thereof.

An antibody producing cell is defined herein as a cell which is capable of producing and/or secreting antibody or a functional equivalent thereof, and/or which is capable of developing into a cell which is capable of producing and/or secreting antibody or a functional equivalent thereof. An antibody producing cell according to the invention is preferably a producer cell which is adapted to commercial antibody production. Preferably, said producer cell is suitable for producing antibodies for use in humans.

A method according to the invention preferably further comprises a step of harvesting, purifying and/or isolating said antibody or functional part, derivative and/or analogue thereof according to the invention. Obtained antibodies or functional equivalents according to the invention are preferably used in human therapy, optionally after additional purifying, isolation or processing steps.

Now that improved Respiratory Syncytial Virus-specific antibodies or functional equivalents according to the invention and nucleic acid sequences and vectors coding therefore have been provided, including human antibodies or functional equivalents, improved prophylactic and/or therapeutic applications have become available. RSV is counteracted by antibodies or functional equivalents according to the invention. An antibody or functional equivalent according to the invention is therefore particularly suitable for use as a medicine or prophylactic agent, optionally in combination with at least one other RSV-specific antibody known in the art. Preferably, antibodies or functional equivalents are used which consist of human sequences, or which have at most 5% of non-human sequences, in order to reduce the chance of adverse side effects when human individuals are treated. An isolated, synthetic or recombinant antibody or a functional part, derivative and/or analogue thereof or a nucleic acid sequence or functional equivalent thereof or a vector or a cell according to the invention for use as a medicament and/or prophylactic agent is therefore also herewith provided. When a nucleic acid or functional equivalent or vector according to the invention is administered, it will be translated *in situ* into an antibody or functional equivalent according to the invention. In a particularly preferred embodiment said antibody comprises antibody AM22, or a functional equivalent thereof. Said medicament or prophylactic agent is preferably used for counteracting or at least in part preventing an infection by RSV. Further provided is therefore an isolated, synthetic or recombinant antibody or a functional part, derivative and/or analogue thereof or a nucleic acid sequence or functional equivalent thereof or vector or cell according to the invention for use as a medicament and/or prophylactic agent for at least in part treating and/or preventing a disorder related to RSV. A medicament that comprises AM22 in combination with at least one other RSV-specific agent, preferably an antibody, known in the art, is particularly advantageous because with such combination a stronger immunogenic response to RSV is obtained and/or higher antibody specificity against RSV is reached. Further provided is therefore a combination of an isolated, synthetic or recombinant antibody or a functional part, derivative and/or analogue thereof or a nucleic acid sequence or functional equivalent thereof or vector or cell according to the invention and another, different RSV-specific agent, preferably an antibody or functional equivalent thereof, for use as a medicament and/or prophylactic agent. A combination according to the invention preferably comprises AM22 and an antibody selected from the group consisting of palivizumab, D25, AM14, AM16 and AM23. As said before, such combination is particularly suitable for at least in part treating or preventing a RSV-related disorder. Further provided is therefore a use of a combination according to the invention for the preparation of a medicament and/or prophylactic agent for at least in part treating and/or preventing a disorder related to RSV. A use of an isolated, synthetic or recombinant antibody or a functional part, derivative and/or analogue thereof or a nucleic acid sequence or functional equivalent thereof or vector or cell according to the invention for the preparation of a medicament and/or prophylactic agent for at least in part treating and/or preventing an RSV-related disorder is therefore also provided, as well as a method for at least in part treating or preventing a RSV-related disorder, the method comprising administering to an individual in need thereof a therapeutically effective amount of an isolated, synthetic or recombinant antibody or a functional part, derivative and/or analogue thereof according to the invention. In one preferred embodiment, a combination with at least one other RSV-specific agent, preferable another RSV specific antibody, is used. Said individual has preferably been diagnosed to be infected by RSV before treatment.

Said antibody preferably comprises antibody AM22, or a functional part thereof. Said at least one other RSV-specific antibody is preferably palivizumab, D25, AM14, AM16 or AM23. Most preferably a combination of AM22 and D25 is used.

In order to at least in part treat or prevent a disorder related to Respiratory Syncytial Virus, an antibody or functional equivalent according to the invention is preferably administered to an individual before an infection has taken place. Alternatively, an antibody or functional equivalent according to the invention is administered when an individual is already infected. Said antibody or functional equivalent is preferably administered to individuals with an increased risk of complications, such as for instance hospitalized individuals and/or individuals with compromised immunity. Also elderly people have an increased risk of RSV infection. Antibodies or functional equivalents according to the invention are preferably administered via one or more injections. Dose ranges of antibodies or functional equivalents according to the invention to be used in the prophylactic or therapeutic applications as described herein before are designed on the basis of rising dose studies in the clinic in clinical trials for which rigorous protocol requirements exist. Typical doses are between 0.1 and 10 mg per kg body weight. For prophylactic or therapeutic application antibodies or functional equivalents according to the invention are typically combined with a pharmaceutically acceptable carrier, diluent and/or excipient. Examples of suitable carriers for instance comprise keyhole limpet haemocyanin (KLH), serum albumin (e.g. BSA or RSA) and ovalbumin. In one preferred embodiment said suitable carrier comprises a solution like for example saline.

In yet another embodiment a nucleic acid or a vector encoding an antibody or functional equivalent according to the invention is used. As already described, upon administration of such nucleic acid or vector, antibodies or functional equivalents are produced by the host's machinery. Produced antibodies or functional equivalents are capable of at least in part preventing and/or counteracting Respiratory Syncytial Virus infection and/or the adverse effects of such infection. A nucleic acid sequence or functional equivalent or a vector according to the invention for use as a medicament and/or prophylactic agent is therefore also herewith provided. Further provided is a use of a nucleic acid sequence or functional equivalent or vector according to the invention for the preparation of a medicament and/or prophylactic agent for at least in part treating and/or preventing a RSV-related disorder.

Further provided is a pharmaceutical composition comprising an isolated, synthetic or recombinant antibody or a functional part, derivative and/or analogue thereof or a nucleic acid sequence or functional equivalent thereof or vector or cell according to the invention and a pharmaceutical acceptable carrier, diluent or excipient. Said pharmaceutical composition is preferably suitable for human use. In one preferred embodiment said antibody is AM22. In a further preferred embodiment said nucleic acid encodes AM22 or a functional equivalent thereof. In one embodiment said pharmaceutical composition further comprises at least one other RSV-specific antibody, preferably palivizumab, D25, AM14, AM16 and/or AM23.

The invention is further explained in the following examples. These examples do not limit the scope of the invention, but merely serve to clarify the invention.

### References

Chen Y, Wiesmann C, Fuh G, Li B, Christinger HW, McKay P, de Vos AM, Lowman HB. Selection and analysis of an optimized anti-VEGF antibody: crystal structure of an affinity-matured Fab in complex with antigen. J Mol Biol. 1999; 293(4):865-81.
Diehl SA, Schmidlin H, Nagasawa M, van Haren SD, Kwakkenbos MJ, Yasuda E, Beaumont T, Scheeren FA, Spits H. STAT3-mediated up-regulation of BLIMP1 Is coordinated with BCL6 down-regulation to control human plasma cell differentiation. J Immunol. 2008; 180(7):4805-15.
Jaleco AC, Stegmann AP, Heemskerk MH, Couwenberg F, Bakker AQ, Weijer K, Spits H. Genetic modification of human B-cell development: B-cell development is inhibited by the dominant negative helix loop helix factor Id3. Blood. 1999; 94(8):2637-46.
Kabat EA, Wu TT. Identical V region amino acid sequences and segments of sequences in antibodies of different specificities. Relative contributions of VH and VL genes, minigenes, and complementarity-determining regions to binding of antibody-combining sites. J Immunol. 1991; 147(5):1709-19.
Kwakkenbos MJ, Diehl SA, Yasuda E, Bakker As, Van Geelen CMM, Lukens MV, Van Bleek GM, Widjojoatmodjo MN, Bogers WMJM, Mei H, Radbruch A, Scheeren FA, Spits H and Beaumont T. Generation of stable monoclonal antibody-producing BCR+ human memory B cells by genetic programming. Nat Med. 2009 In press.
Shvarts A, Brummelkamp TR, Scheeren F, Koh E, Daley GQ, Spits H, Bernards R. A senescence rescue screen identifies BCL6 as an inhibitor of antiproliferative p19(ARF)-p53 signaling. Genes Dev. 2002; 16(6):681-6.
Scheeren FA, Naspetti M, Diehl S, Schotte R, Nagasawa M, Wijnands E,
Gimeno R, Vyth-Dreese FA, Blom B, Spits H. STAT5 regulates the self-renewal capacity and differentiation of human memory B cells and controls Bcl-6 expression. Nat Immunol. 2005; 6(3):303-13.
Ternette N, Tippler B, Uberla K, Grunwald T. Immunogenicity and efficacy of codon optimized DNA vaccines encoding the F-protein of respiratory syncytial virus. Vaccine. 2007; 25(41):7271-9.
   US 5,624,821
   US 5,739,277
   US 6,277,375
   US 6,737,056
   US 6,821,505
   US 7,083,784
   US 7,317,091
   US 7,217,797
   US 7,276,585
   US 7,355,008
   US 2002/0147311
   US 2004/0002587
   US 2005/0215768
   US 2007/0135620
   US 2007/0224188
   US 2008/0089892
   WO 94/29351
   WO 99/58572
   WO 2008/147196

### Figure legends

Figure 1. AM22, a novel fully human monoclonal antibody, neutralizes the RSV A2 virus very efficiently on Hep2 cells compared to palivizumab.
Figure 2. AM22 heavy and light chain nucleotide sequences and amino acid sequences.
Figure 3. Human anti-RSV monoclonal antibodies recognize conformational epitopes on the Fusion (F) protein of RSV as determined by ELISA and FACS staining. (A) shows binding of the antibodies to EL-4 cells infected with Vesicular Stomatitis Virus (VSV) which was pseudotyped with the RSV F or RSV G protein. (B) shows binding of the anti-RSV F antibodies to ELISA plates that were coated with a lysate of RSV infected HEp2 (left panel) or with Ni-NTA HisSorp Plates (Qiagen) coated with recombinant HIS tagged F protein (right panel). Antibody binding to the F protein is detected with HRP-conjugated IgG detection antibody (dilutions 1:2500, Jackson). (C) shows binding of the recombinant RSV F Long strain containing a poly HIS tag to the original B cell clones, binding to the BCR is detected with an anti-pentaHIS antibody. In (D) we show the intracellular binding of the human anti-RSV antibodies to 293T cells transfected with a recombinant RSV F construct containing a trimerization domain (ILZ domain).
Figure 4. RSV challenge in Cotton rats prophylatically treated with human immunoglobulins. (A) shows the retrieval of human IgG1 from serum at day 1 and day 5 after intramuscular administration of indicated doses of antibodies in Cotton rats. (B) shows the RSV load in the lungs of Cotton rats treated with the indicated antibodies 24 hours before infection with RSV-X. RSV load was determined 5 days after infection with TCID₅₀ culture. Experiments were performed twice with four to six individual animals per treatment group. Lung pathology was studied in the same animal groups (C), indicated is the average lungpathology of AM22 and palivizumab, see also table 2.

### Examples

### Example 1. B cell culture, immortalization and selection.

### Methods

B cells were immortalized and cultured as described before (Scheeren FA, et al. (2005) Nat Immunol 6: 303-313; Diehl SA, et al. (2008) J Immunol 180: 4805-4815 and Kwakkenbos MJ, et al. (2009) Nat Med in press). In brief, we isolated B cells from peripheral blood (Sanquin, Amsterdam, The Netherlands) by Ficoll separation, CD22 MACS microbeads (Miltenyi Biotech) and subsequently cell sorting for CD19⁺CD3⁻CD27⁺IgM⁻IgA⁻ (IgG memory cells) on a FACSAria (Becton Dickinson). The use of these tissues was approved by the medical ethical committees of the institution and was contingent on informed consent. Retroviral transduced B cells, were maintained at 2x10⁵ cells ml⁻¹ in IMDM supplemented with recombinant mouse IL-21 (25 ng ml⁻¹, R&D systems) and co-cultured with γ-irradiated (50Gy) mouse L cell fibroblasts stably expressing CD40L (CD40L-L cells, 10⁵ cells ml⁻¹) for 36 hours. The BCL6 and Bcl-xL retroviral constructs were described previously (Shvarts A, et al. (2002) Genes Dev 16: 681-686 and Jaleco AC, et al. (1999) Blood 94: 2637-2646)) and were cloned into the LZRS retroviral vector and transfected in Phoenix packaging cells as described before and added to the stimulated B cells (Shvarts A, et al. (2002) Genes Dev 16: 681-686 and Scheeren FA, et al. (2005) Nat Immunol 6: 303-313). Transduced B cells were maintained in IMDM, in the presence of recombinant IL-21 and CD40L-L cells for prolonged periods of time. Given the relatively high amounts of secreted antibodies by BCL6+Bcl-xL-transduced B cells we examined whether we could select antigen-specific B cells on the basis of secretion of specific antibody. BCL6+Bcl-xL transduced memory B cells of a healthy donor were seeded at 100 cells/well and expanded with CD40L-L cells and IL-21. After 2 weeks of culture, supernatants were harvested and screened for the presence of RSV-neutralizing antibodies in a microneutralization experiment. Of 384 cultures (100 cells/well), 31 prevented RSV A2 infection of HEp2 cells. Besides the four microcultures from which D25, AM14, AM16, and AM23 were subcloned by limiting dilution, we next obtained AM22. AM22 has a median half maximum inhibitory concentrations (IC₅₀) against the RSV-A2 virus of 1.15 ng ml⁻¹ (Fig. 1).

To obtain the sequence of the heavy and light chain of the immunoglobulin locus of AM22, we isolated total RNA with the RNeasy® mini kit (Qiagen), generated cDNA, performed PCR and cloned the heavy and light chain variable regions into the pCR2.1 TA cloning vector (Invitrogen). To rule out reverse transcriptase or DNA polymerase induced mutations, we performed several independent cloning experiments. To produce recombinant AM22 mAb we cloned AM22 heavy and light variable regions in frame with human IgG1 and Kappa constant regions into a pcDNA3.1 (Invitrogen) based vector and transiently transfected 293T cells. We purified recombinant AM22 from the culture supernatant with Protein A.

### Results

Previously we developed four potent conformational dependent anti-RSV antibodies, named AM14, AM16, AM23 and D25. These antibodies have been described in WO 2008/147196 and by Kwakkenbos MJ et al. (2009) Nat Med in press. From the same donor we also discovered AM22, which recognized the RSV virus even more efficiently compared to the other antibodies as is evident from an IC₅₀ of 1.15 ng ml⁻¹ against the RSV A2 virus (table 1 and figure 1). The amino acid sequence of the VH and VL chain of AM22 revealed that this antibody is different from the other antibodies (figure 2).

### Example 2. In vitro binding experiments.

To further determine the antigen specificity of the AM22 antibody we performed in *vitro* binding experiments to reveal whether the protein recognized the RSV F or G protein and in what type of conformation.

### Methods

### (1) FACS staining of RSV G or F protein

Virus stock of wild type and recombinant Vesicular Stomatitis Virus (VSV) expressing RSV-G protein (VSV-G) or RSV-F protein (VSV-F) (experiments were performed by M. Lukens at WKZ, Utrecht and VSV viruses were kindly provided by J.S. Kahn and J.K Rose, Yale University School of Medicine) were prepared on BHK cells grown in DMEM containing 5 % FCS, penicillin/streptomycin and 50 µM 2-mercapto-ethanol. VSV infection was performed on EL-4 cells that were cultured in Iscove's Modified Dulbecco's medium (IMDM, Gibco, Invitrogen) supplemented with 5 % FCS, penicillin/streptomycin and 50 µM 2-mercapto-ethanol. EL-4 cells infected with the VSV virus variants were incubated with recombinant antibody and subsequently stained with mouse-anti-human PE (figure 3A).

### (2) RSV ELISA

Plates were coated with lysate of RSV infected HEp-2 cells in PBS for 1 hour at 37 °C or o/n at 4 °C and washed in ELISA wash buffer (PBS, 0.5 % Tween-20). Plates were blocked by incubation with 4 % milk in PBS, before the anti-RSV antibodies or polyclonal goat anti-RSV (Biodesign) in combination with enzyme-conjugated anti-IgG antibodies were added (dilutions 1:2500 for HRP-conjugated anti-IgG (Jackson). TMB substrate/stop solution (Biosource) was used for development of the ELISAs (figure 3B left panel). In addition to the lysate of RSV-A2 infected HEp-2 cells, HIS-tagged F protein from RSV Long strain (kindly provided by Frank Coenjaerts, UMCU, Utrecht based on Ternette N, et al, (2007) Vaccine 25: 7271-7279) was used to coat Ni-NTA HisSorp Plates (Qiagen) (figure 3B right panel). The binding of the RSV F antibodies was detected with HRP-conjugated IgG detection antibody (dilutions 1:2500, Jackson). In another setting the original B cell clones were used for FACS analysis (figure 3C). The B cells were incubated with the recombinant HIS tagged F protein and binding to the BCR was detected with a labeled anti-penta HIS antibody ALEXA fluor 647 (Qiagen).

### (3) RSV trimers

In addition to the recombinant RSV long strain derived F protein we created RSV A2 F trimers by inserting the open reading frame of F into a construct which is fused to an isoleucine zipper domain followed by 8 HIS repeats (ILZ-8xHIS). Protein constructs were transiently expressed in 293T cells and detected by using an intracellular staining protocol using the Fix Perm kit of BD (figure 3D).

### Results

All antibodies recognized the RSV-F protein when expressed by recombinant VSV (figure 3A). Furthermore, except for AM16, recognition was dependent on the presence of conformational epitopes in the RSV-F protein since they did not recognize a lysate of RSV infected HEp-2 cells (figure 3B left panel). Also purified HIS-tagged recombinant RSV-F Long strain protein was not recognized by D25, AM14, AM22 and AM23 when tested in a direct ELISA (Figure 3B right panel). However when the original stable BCR expressing B cell lines were incubated with this non-purified culture supernatant of the HIS tagged RSV-F protein we did observe binding to the B cell clones AM16, AM23 and less strong to D25 (figure 3C). The protein in this non-purified culture supernatant possibly contains a fraction of RSV F trimers that do bind the BCR but are only captured as monomers on the HisSorp Plates. However still no RSV-F protein capture was observed to the BCRs of the AM14 and the AM22 B cell lines (figure 3C). Thus AM14 and AM22 bind to different epitopes. Possibly a low percentage of protein F homotrimers or dimers was present in the untreated culture supernatant of the F protein producing cell line. These more native F conformations may express the epitopes recognized by AM23 and D25, which were lost in the denaturizing conditions of the ELISA procedures. Interestingly when we performed an intracellular staining on 293T cells transfected with the RSV trimerization construct containing the ILZ-8xHIS sequence we found that AM22 did recognize RSV F (figure 3D), however AM14 still did not recognize this protein structure. Thus AM22 is unique since it recognizes a conformation of the RSV-F protein that is not present in denaturing conditions and thus is not present in the monomeric form of the protein. Only when the RSV F proteins are forced to stay together and form trimers then we can detect F binding by the AM22 antibody, a conformation that is present on virus particles and thus explain the high neutralizing potency of AM22.

### Example 3. In vivo potency experiments.

To study the *in vivo* potency of the AM22 monoclonal antibody we performed Cotton rat experiments.

### Methods

Pathogen free 7-9 week-old Cotton rats (*Sigmodon hispidus,* Harlan Laboratories, Nederland) were anesthetized with isoflurane and given 0.1 ml of purified antibody by intramuscular (i.m.) injection at doses of 2.0 or 0.4 mg kg⁻¹ for the control antibody, palivizumab, AM22, AM23 and D25, while AM14 was administered at 0.4 and 0.1 mg kg⁻¹. Twenty-four hours later, animals were anesthetized, bled for serum hIgG determination and challenged by intranasal instillation of 10⁶ TCID₅₀ RSV-X (100 µl). Five days later animals were sacrificed and their lungs were harvested. Lung virus titers were determined and the lowest limit of detection was 2.1 log₁₀ TCID₅₀ g⁻¹. The Animal Experiment Committee of the Netherlands Vaccine Institute approved all procedures involving Cotton rats.

### Results

The anti-RSV antibody panel, except AM16, was tested in Cotton rats. Animals were prophylactically treated with 2.0 or 0.4 mg kg⁻¹ of monoclonal antibodies before RSV-X, a primary RSV A isolate, was intra-nasally administered. Due to relatively low antibody production, the AM14 antibody was administered at 0.4 and 0.1 mg kg⁻¹. The level of recovered human IgG from Cotton rat sera at day 1 (day of virus inoculation) and day 5 (day of sacrifice) was in the same range for all antibodies and the decline of antibodies in time was comparable (figure 4A).

The retrieval of RSV virus from the lungs of sacrificed animals was strongly reduced in all animal groups treated with 2.0 mg/kg of immunoglobulin compared to the control group (figure 4B). Animals treated with 0.4 mg kg⁻¹ palivizumab and AM23 showed significant virus replication, while in the AM14 and D25 groups one out of 6 animals showed detectable virus replication. No virus could be retrieved from animals treated with AM22. These results demonstrate that AM22, that specifically recognized conformational epitopes on the RSV F protein, harbors strong *in vivo* neutralizing capacities.

### Analysis of lung pathology of RSV challenged Cotton rats

From each Cotton rat at day 5 after i.n. RSV infection the left lung was removed and fixed with formalin. Lung damage was classified between 0-5 for three individual markers: 1) hypertrophy of bronchus and bronchioli epithelia, 2) inflammation surrounding bronchus and bronchioli (peribronchiolitis) and 3) inflammation in the alveoli (alveolitis). The average sum of the scores of all animals in one group created the pathology index (maximum score 15) (figure 4c, table 2). Lung pathology after RSV infection was significantly reduced in animal groups treated with high doses of immunoglobulin (2 mg kg⁻¹)(table 3). However only in the AM14, AM22 and AM23 groups the pathology was significantly reduced at lower concentrations (0.4 mg kg⁻¹). While a complete absence of pathology was seen in 3 out of 5 animals treated with AM22 and AM23 at 2 mg kg⁻¹, at 0.4 mg kg⁻¹ complete protection was detected in 2 or 1 out of 6 animals in the AM14 and AM22 groups respectively (table 3).

When combining the results of examples 1, 2 and 3 it is concluded that AM22 has certain advantages. AM22 demonstrates the lowest IC₅₀ value, meaning that with a lower amount of AM22 similar prophylactic or therapeutic effects are achieved as compared with other antibodies. AM22 recognizes a different epitope of the RSV F protein than the other antibodies and therefore can be used in combination with one of the other antibodies to achieve a stronger immunogenic response to RSV and higher antibody specificity against RSV. Finally, treatment of Cotton rats with AM22 resulted in nearly complete inhibition of virus replication with potential complete absence of pathology in these Cotton rats.

### Example 4: Affinity of AM22 for the RSV F protein

Determining the affinity constant and specificity of binding between the RSV F protein and AM22 is preferred to establish the prophylactic, therapeutic and diagnostic value of the antibody. This is a challenging feature since the antibody affinity has to be determined for an oligomeric protein structure. Usually affinity constants are determined against immobilized agents that are captured on a chip. However protein F captured on a chip would not be recognized by the AM14, AM22, AM23 and D25 antibodies.

### Method

"Binding affinity" generally refers to the strength of the sum total of the noncovalent interactions between a single binding site of a molecule (e.g., an antibody) and its binding partner (e.g., an antigen). The affinity of a molecule X for its partner Y can generally be represented by the equilibrium dissociation constant (Kd), which is calculated as the ratio k_{off}/kₒₙ. Affinity can be measured by common methods known in the art, such as a surface plasmon resonance (SPR) assay. Affinities (KD), on-rates (ka) and off-rates (kd) will be measured by SPR analysis with the IBIS-iSPR instrument at IBIS Technologies BV (Hengelo, the Netherlands). Briefly, anti-RSV antibodies are immobilized and purified RSV F protein (containing penta-HIS) is diluted and rate and affinity constants are measured by injection of at least three serial dilutions of the protein.

Another set up in the IMIS-iSPR machine is to immobilize 1) an anti-penta HIS antibody on which the F-penta HIS protein is coupled or 2) F-penta HIS protein is directly immobilized on the chip and then samples on the chip are incubated with the AIMM antibodies to determine the affinity constants.

**Table 1. RSV neutralizing activity of purified IgGs.**

| | RSV A2^{a} |
|---|---|
| palivizumab | 152 |
| D25 | 1.28 |
| AM14 | 2.09 |
| AM16 | 78.7 |
| AM22 | 1.15 |
| AM23 | 4.18 |

The IC₅₀ (ng/ml) values of the selected anti-RSV IgGs were determined with standard TCID₅₀ culture assay on HEp2 cells with the RSV A2 virus.

**Table 2. Cumulative pathology score in Cotton rats.**

| antibody | mg/kg | Pathology score (SEM) | P values |
|---|---|---|---|
| Palivizumab | 2.0 | 3.20 (0.4) | 0.0005 |
| Palivizumab | 0.4 | 5.40 (0.8) | < 0.05 |
| D25 | 2.0 | 3.40 (0.4) | 0.0005 |
| D25 | 0.4 | 5.83 (1.0) | 0.05 |
| AM14 | 0.4 | 4.20 (0.8) | 0.05 |
| AM14 | 0.1 | 3.67 (1.2) | 0.05 |
| AM22 | 2.0 | 2.75 (0.8) | 0.0005 |
| AM22 | 0.4 | 4.00 (0.9) | < 0.05 |
| AM23 | 2.0 | 2.40 (0.2) | 0.0005 |
| AM23 | 0.4 | 3.50(0.9) | < 0.05 |
| Ctrl IgG1 | 2.0 | 8.80 (1.1) | N.A. |
| MOCK | N.A. | 1.80 (0.4) | 0.0005 |

Cumulative pathology score of the lungs of Cotton rats, treated 24 hour before RSV infection with the indicated antibodies. Lung specimens obtained 5 days after infection were evaluated by a pathologist randomly. Lungpathology was classified with scores between 0-5 for three individual markers: 1) hyperthropy of bronchus and bronchioli epithelia, 2) peribronchiolitis and 3) alveolitis. Average pathology score (maximum score 15) with standard error of the mean (SEM) and statistical differences in relation to Ctrl IgG1 group was calculated with the 2-sided Wilcoxon test. Experiments were performed twice with four to six individual animals per treatment group. N.A. not applicable.

**Table 3. Prevention of pathology in Cotton rats.**

| antibody | mg/kg | Significant reduction in pathology | No pathology |
|---|---|---|---|
| Palivizumab | 2 | 5/5 | 1/5 |
| Palivizumab | 0.4 | 3/5 | 0/6 |
| D25 | 2 | 5/5 | 0/5 |
| D25 | 0.4 | 2/6 | 0/6 |
| AM14 | 0.4 | 5/6 | 2/6 |
| AM14 | 0.1 | 4/5 | 0/5 |
| AM22 | 2 | 5/5 | 3/5 |
| AM22 | 0.4 | 5/6 | 1/6 |
| AM23 | 2 | 5/5 | 3/5 |
| AM23 | 0.4 | 5/6 | 0/6 |

Number of animals with significant reduction in / or absence of lung pathology at day 5 during RSV-X infection. Experiments were performed twice with four to six individual animals per treatment group.

## Claims

1. An isolated, synthetic or recombinant antibody or a functional part thereof which is capable of specifically binding Respiratory Syncytial Virus (RSV) F protein and which comprises:
a heavy chain complementarity determining region (CDR) 1 sequence comprising the sequence KLSIH,
a heavy chain CDR2 sequence comprising the sequence GYEGEVDEIFYAQKFQH,
a heavy chain CDR3 sequence comprising the sequence LGVTVTEAGLGIDDY,
a light chain CDR1 sequence comprising the sequence RASQIVSRNHLA,
a light chain CDR2 sequence comprising the sequence GASSRAT, and
a light chain CDR3 sequence comprising the sequence LSSDSSI.

2. The antibody or functional part thereof according to claim 1, wherein (a) the heavy chain sequence comprises a sequence which is at least 90% identical to the sequence 
QVQLVQSGAEVKKPGATVKVSCKISGHTLIKLSIHWVRQAPGKGLEWMGG
YEGEVDEIFYAQKFQHRLTVIADTATDTVYMELGRLTSDDTAVYFCGTLGV
TVTEAGLGIDDYWGQGTLVTVSS (Figure 2);
(b) the light chain sequence comprises a sequence which is at least 90% identical to the sequence 
EIVLTQSPGTLSLSPGERATLSCRASQIVSRNHLAWYQQKPGQAPRLLIFGA
SSRATGIPVRFSGSGSGTDFTLTINGLAPEDFAVYYCLSSDSSIFTFGPGTKV
DFK (Figure 2);
(c) the heavy chain sequence comprises the sequence 
QVQLVQSGAEVKKPGATVKVSCKISGHTLIKLSIHWVRQAPGKGLEWMGG
YEGEVDEIFYAQKFQHRLTVIADTATDTVYMELGRLTSDDTAVYFCGTLGV
TVTEAGLGIDDYWGQGTLVTVSS (Figure 2);
(d) the light chain sequence comprises the sequence 
EIVLTQSPGTLSLSPGERATLSCRASQIVSRNHLAWYQQKPGQAPRLLIFGA
SSRATGIPVRFSGSGSGTDFTLTINGLAPEDFAVYYCLSSDSSIFTFGPGTKVDFK
(Figure 2);
(e) the heavy chain sequence comprises a sequence which is at least 90% identical to the sequence
QVQLVQSGAEVKKPGATVKVSCKISGHTLIKLSIHWVRQAPGKGLEWMGG YEGEVDEIFYAQKFQHRLTVIADTATDTVYMELGRLTSDDTAVYFCGTLGV TVTEAGLGIDDYWGQGTLVTVSS (Figure 2) and the light chain sequence comprises a sequence which is at least 90% identical to the sequence 
EIVLTQSPGTLSLSPGERATLSCRASQIVSRNHLAWYQQKPGQAPRLLIFGA
SSRATGIPVRFSGSGSGTDFTLTINGLAPEDFAVYYCLSSDSSIFTFGPGTKVDFK
(Figure 2);
(f) a heavy chain sequence comprises a sequence which is at least 90% identical to the sequence
QVQLVQSGAEVKKPGATVKVSCKISGHTLIKLSIHWVRQAPGKGLEWMGG YEGEVDEIFYAQKFQHRLTVIADTATDTVYMELGRLTSDDTAVYFCGTLGV TVTEAGLGIDDYWGQGTLVTVSS (Figure 2) and the light chain sequence comprises the sequence 
EIVLTQSPGTLSLSPGERATLSCRASQIVSRNHLAWYQQKPGQAPRLLIFGA
SSRATGIPVRFSGSGSGTDFTLTINGLAPEDFAVYYCLSSDSSIFTFGPGTKVDFK
(Figure 2);
(g) the heavy chain sequence comprises the sequence
QVQLVQSGAEVKKPGATVKVSCKISGHTLIKLSIHWVRQAPGKGLEWMGG YEGEVDEIFYAQKFQHRLTVIADTATDTVYMELGRLTSDDTAVYFCGTLGV TVTEAGLGIDDYWGQGTLVTVSS (Figure 2) and the light chain sequence comprises a sequence which is at least 90% identical to the sequence 
EIVLTQSPGTLSLSPGERATLSCRASQIVSRNHLAWYQQKPGQAPRLLIFGA
SSRATGIPVRFSGSGSGTDFTLTINGLAPEDFAVYYCLSSDSSIFTFGPGTKVD
FK (Figure 2); or
(h) the heavy chain sequence comprises the sequence
QVQLVQSGAEVKKPGATVKVSCKISGHTLIKLSIHWVRQAPGKGLEWMGG YEGEVDEIFYAQKFQHRLTVIADTATDTVYMELGRLTSDDTAVYFCGTLGV TVTEAGLGIDDYWGQGTLVTVSS (Figure 2) and the light chain sequence comprises the sequence

EIVLTQSPGTLSLSPGERATLSCRASQIVSRNHLAWYQQKPGQAPRLLIFGA
SSRATGIPVRFSGSGSGTDFTLTINGLAPEDFAVYYCLSSDSSIFTFGPGTKVDFK
(Figure 2).

3. The antibody or functional part thereof according to claim 1 or 2, which is conjugated to PEG or Albumin, and/or which comprises:
- a salvage receptor binding epitope, and/or;
- modified amino acid residues identified as involved in the interaction between and Fc and an FcRN receptor, and/or
- non naturally occurring amino acid residues.

4. The antibody or functional part thereof according to any one of claims 1 to 3, comprising an Fc region which comprises a modification at one or more positions selected from the group consisting 234, 235, 236, 237, 238, 239, 240, 241, 243, 244, 245, 247, 251, 252, 254, 255, 256, 262, 263, 264, 265, 266, 267, 268, 269, 279, 280, 284, 292, 296, 297, 298, 299, 305, 313, 316, 325, 326, 327, 328, 329, 330, 331, 332, 333, 334, 339, 341, 343, 370, 373, 378, 392, 416, 419, 421, 440 and 443 as numbered by the EU index as set forth in Kabat et al, said modification comprising an amino acid substitution, and/or an amino acid insertion, and/or an amino acid deletion.

5. The antibody or functional part thereof according to any one of claims 1 to 4 wherein said antibody or functional part thereof has a dissociation constant (Kd) of less than 1 x 10⁻²M, 1 x 10⁻³M, 1 x 10⁻⁴M. 1 x 10⁻⁵M, 1 x 10⁻⁶M, 1 x 10⁻⁷M, 1 x 10⁻⁸M, 1 x 10⁻⁹M, 1 x 10-¹⁰M, 1 x 10⁻¹¹M, 1 x 10⁻¹²M, 1 x 10⁻¹³M, 1 x 10⁻¹⁴M or less than 1 x 10⁻¹⁵M.

6. The antibody or functional part thereof according to any one of claims 1 to 5 which is a human antibody.

7. The antibody or functional part thereof according to any one of claims 1 to 5 wherein the functional part thereof is a single chain antibody, single variable fragment (sFv), Fab fragrant or F(ab')2 fragment.

8. An isolated, synthetic or recombinant nucleic acid sequence, or a functional equivalent thereof, encoding the antibody or functional part thereof according to any one of claims 1 to 4, 6 or 7.

9. The nucleic acid sequence or functional equivalent thereof according to claim 8, which comprises the sequences

aaattatccattcac,
ggttatgagggtgaggtcgatgagattttctacgcacagaagttccagcac,
ctaggtgtgacagtgactgaggctggactggggatcgatgactac,
agggccagtcagattgttagcaggaaccacttagcc, ggtgcgtccagtcgggccact and
ctgtcctctgattcctccata.

10. The nucleic acid sequence or functional equivalent thereof according to claim 8 which comprises the nucleotide sequence of the heavy chain and/or light chain sequences in figure 2.

11. A vector comprising a nucleic acid sequence or functional equivalent according to any one of claims 8 to 10.

12. An isolated or recombinant cell comprising or engineered to express the nucleic acid sequence or functional equivalent according to any one of claims 8 to 10, and/or the vector according to claim 11.

13. A combination comprising:
(a) the antibody or functional part thereof, or nucleic acid sequence or functional equivalent, or vector, or cell according to any one of claims 1 to 12 and
(b) a different RSV-specific agent, preferably an antibody, wherein the antibody is preferably, Palivizumab, and/or AM14 and/or AM16, and/or AM23, and/or D25.

14. A pharmaceutical composition comprising:
(a) the antibody or functional part thereof, or a nucleic acid sequence or functional equivalent thereof, or vector, or cell, or combination according to any one of claims 1 to 13 and
(b) a pharmaceutical acceptable carrier, diluent or excipient.

15. The antibody or functional part thereof, or a nucleic acid sequence or functional equivalent, or vector, or cell, or combination or pharmaceutical composition according to any one of claims 1 to 14 for use as a medicament and/or prophylactic agent for at least in part treating and/or preventing a RSV-related disorder.

16. A method for producing an antibody or functional part thereof according to claims 1 to 7 comprising providing a cell with a nucleic acid sequence or functional equivalent thereof according to any one of claims 8 to 10 or a vector according to claim 11 and allowing said cell to translate said nucleic acid sequence or functional equivalent thereof or said vector, thereby producing said antibody or functional part thereof.

17. The method according to claim 16 further comprising harvesting, purifying and/or isolating said antibody or functional part thereof.

## Patentansprüche

1. Isolierter, synthetischer oder rekombinanter Antikörper oder funktioneller Teil davon, der zum spezifischen Binden von Respiratorisches-Synzytial-Virus- (RSV-) F-Protein fähig ist und der umfasst:
eine Schwerketten-komplementaritätsbestimmende-Region- (-CDR-) 1-Sequenz, umfassend die Sequenz KLSIH,
eine Schwerketten-CDR2-Sequenz, umfassend die Sequenz GYEGEVDEIFYAQKFQH,
eine Schwerketten-CDR3-Sequenz, umfassend die Sequenz LGVTVTEAGLGIDDY,
eine Leichtketten-CDR1-Sequenz, umfassend die Sequenz RASQIVSRNHLA,
eine Leichtketten-CDR2-Sequenz, umfassend die Sequenz GASSRAT, und
eine Leichtketten-CDR3-Sequenz, umfassend die Sequenz LSSDSSI.

2. Antikörper oder funktioneller Teil davon nach Anspruch 1, wobei
(a) die Schwerkettensequenz eine Sequenz umfasst, die zu mindestens 90% identisch ist mit der Sequenz 
QVQLVQSGAEVKKPGATVKVSCKISGHTLIKLSIHWVRQAPGKGLEWMGG
YEGEVDEIFYAQKFQHRLTVIADTATDTVYMELGRLTSDDTAVYFCGTLGV
TVTEAGLGIDDYWGQGTLVTVSS (Figur 2);
(b) die Leichtkettensequenz eine Sequenz umfasst, die zu mindestens 90% identisch ist mit der Sequenz 
EIVLTQSPGTLSLSPGERATLSCRASQIVSRNHLAWYQQKPGQAPRLLIFGA
SSRATGIPVRFSGSGSGTDFTLTINGLAPEDFAVYYCLSSDSSIFTFGPGTKV
DFK (Figur 2);
(c) die Schwerkettensequenz die Sequenz 
QVQLVQSGAEVKKPGATVKVSCKISGHTLIKLSIHWVRQAPGKGLEWMGG
YEGEVDEIFYAQKFQHRLTVIADTATDTVYMELGRLTSDDTAVYFCGTLGV
TVTEAGLGIDDYWGQGTLVTVSS (Figur 2)
 umfasst;
(d) die Leichtkettensequenz die Sequenz 
EIVLTQSPGTLSLSPGERATLSCRASQIVSRNHLAWYQQKPGQAPRLLIFGA
SSRATGIPVRFSGSGSGTDFTLTINGLAPEDFAVYYCLSSDSSIFTFGPGTKVDFK
(Figur 2)
 umfasst;
(e) die Schwerkettensequenz eine Sequenz umfasst, die zu mindestens 90% identisch ist mit der Sequenz 
QVQLVQSGAEVKKPGATVKVSCKISGHTLIKLSIHWVRQAPGKGLEWMGG
YEGEVDEIFYAQKFQHRLTVIADTATDTVYMELGRLTSDDTAVYFCGTLGV
TVTEAGLGIDDYWGQGTLVTVSS (Figur 2),
 und die Leichtkettensequenz eine Sequenz umfasst, die zu mindestens 90% identisch ist mit der Sequenz 
EIVLTQSPGTLSLSPGERATLSCRASQIVSRNHLAWYQQKPGQAPRLLIFGA
SSRATGIPVRFSGSGSGTDFTLTINGLAPEDFAVYYCLSSDSSIFTFGPGTKVDFK
(Figur 2);
(f) eine Schwerkettensequenz eine Sequenz umfasst, die zu mindestens 90% identisch ist mit der Sequenz 
QVQLVQSGAEVKKPGATVKVSCKISGHTLIKLSIHWVRQAPGKGLEWMGG
YEGEVDEIFYAQKFQHRLTVIADTATDTVYMELGRLTSDDTAVYFCGTLGV
TVTEAGLGIDDYWGQGTLVTVSS (Figur 2),
 und die Leichtkettensequenz die Sequenz 
EIVLTQSPGTLSLSPGERATLSCRASQIVSRNHLAWYQQKPGQAPRLLIFGA
SSRATGIPVRFSGSGSGTDFTLTINGLAPEDFAVYYCLSSDSSIFTFGPGTKVDFK
(Figur 2)
 umfasst;
(g) die Schwerkettensequenz die Sequenz 
QVQLVQSGAEVKKPGATVKVSCKISGHTLIKLSIHWVRQAPGKGLEWMGG
YEGEVDEIFYAQKFQHRLTVIADTATDTVYMELGRLTSDDTAVYFCGTLGV
TVTEAGLGIDDYWGQGTLVTVSS (Figur 2)
 umfasst
und die Leichtkettensequenz eine Sequenz umfasst, die zu mindestens 90% identisch ist mit der Sequenz 
EIVLTQSPGTLSLSPGERATLSCRASQIVSRNHLAWYQQKPGQAPRLLIFGA
SSRATGIPVRFSGSGSGTDFTLTINGLAPEDFAVYYCLSSDSSIFTFGPGTKVD
FK (Figur 2); oder
(h) die Schwerkettensequenz die Sequenz 
QVQLVQSGAEVKKPGATVKVSCKISGHTLIKLSIHWVRQAPGKGLEWMGG
YEGEVDEIFYAQKFQHRLTVIADTATDTVYMELGRLTSDDTAVYFCGTLGV
TVTEAGLGIDDYWGQGTLVTVSS (Figur 2)
 umfasst
und die Leichtkettensequenz die Sequenz 
EIVLTQSPGTLSLSPGERATLSCRASQIVSRNHLAWYQQKPGQAPRLLIFGA
SSRATGIPVRFSGSGSGTDFTLTINGLAPEDFAVYYCLSSDSSIFTFGPGTKVDFK
(Figur 2)
 umfasst.

3. Antikörper oder funktioneller Teil davon nach Anspruch 1 oder 2, der an PEG oder Albumin konjugiert ist, und/oder der umfasst:
- ein Salvage-Rezeptor-Bindungsepitop, und/oder
- modifizierte Aminosäurereste, identifiziert als involviert in die Wechselwirkung zwischen Fc und einem FcRN-Rezeptor, und/oder
- nicht natürlich vorkommende Aminosäurereste.

4. Antikörper oder funktioneller Teil davon nach einem der Ansprüche 1 bis 3, umfassend eine Fc-Region, die eine Modifikation an einer oder mehreren Positionen umfasst, ausgewählt aus der Gruppe, bestehend aus 234, 235, 236, 237, 238, 239, 240, 241, 243, 244, 245, 247, 251, 252, 254, 255, 256, 262, 263, 264, 265, 266, 267, 268, 269, 279, 280, 284, 292, 296, 297, 298, 299, 305, 313, 316, 325, 326, 327, 328, 329, 330, 331, 332, 333, 334, 339, 341, 343, 370, 373, 378, 392, 416, 419, 421, 440 und 443 wie nummeriert nach dem EU-Index gemäß Darlegung in Kabat et al., wobei die Modifikation eine Aminosäuresubstitution, und/oder eine Aminosäureinsertion, und/oder eine Aminosäuredeletion umfasst.

5. Antikörper oder funktioneller Teil davon nach einem der Ansprüche 1 bis 4, wobei der Antikörper oder der funktionelle Teil davon eine Dissoziationskonstante (Kd) aufweist von weniger als 1 x 10⁻²M, 1 x 10⁻³M, 1 x 10⁻⁴M, 1 x 10⁻⁵M, 1 x 10⁻⁶M, 1 x 10⁻⁷M, 1 x 10⁻⁸M, 1 x 10⁻⁹M, 1 x 10⁻¹⁰M, 1 x 10⁻¹¹M, 1 x 10⁻¹²M, 1 x 10⁻¹³M, 1 x 10⁻¹⁴M oder weniger als 1 x 10⁻¹⁵M.

6. Antikörper oder funktioneller Teil davon nach einem der Ansprüche 1 bis 5, der ein menschlicher Antikörper ist.

7. Antikörper oder funktioneller Teil davon nach einem der Ansprüche 1 bis 5, wobei der funktionelle Teil davon ein Einzelkettenantikörper, Einzel-variables-Fragment (sFv), Fab-Fragment oder F(ab')2-Fragment ist.

8. Isolierte, synthetische oder rekombinante Nukleinsäuresequenz, oder funktionelles Äquivalent davon, codierend für den Antikörper oder den funktionellen Teil davon nach einem der Ansprüche 1 bis 4, 6 oder 7.

9. Nukleinsäuresequenz oder funktionelles Äquivalent davon nach Anspruch 8, die bzw. das die Sequenzen 
aaattatccattcac,
ggttatgagggtgaggtcgatgagattttctacgcacagaagttccagcac,
ctaggtgtgacagtgactgaggctggactggggatcgatgactac,
agggccagtcagattgttagcaggaaccacttagcc, ggtgcgtccagtcgggccact und
ctgtcctctgattcctccata
 umfasst.

10. Nukleinsäuresequenz oder funktionelles Äquivalent davon nach Anspruch 8, die bzw. das die Nukleotidsequenz der Schwerketten- und/oder Leichtkettensequenzen in Figur 2 umfasst.

11. Vektor, umfassend eine Nukleinsäuresequenz oder ein funktionelles Äquivalent nach einem der Ansprüche 8 bis 10.

12. Isolierte oder rekombinante Zelle, mit oder konstruiert zum Exprimieren von der Nukleinsäuresequenz oder dem funktionellen Äquivalent nach einem der Ansprüche 8 bis 10, und/oder dem Vektor nach Anspruch 11.

13. Kombination, umfassend:
(a) den Antikörper oder den funktionellen Teil davon, oder die Nukleinsäuresequenz oder das funktionelle Äquivalent, oder den Vektor, oder die Zelle nach einem der Ansprüche 1 bis 12 und
(b) ein anderes RSV-spezifisches Mittel, vorzugsweise einen Antikörper, wobei der Antikörper bevorzugt Palivizumab, und/oder AM 14, und/oder AM16, und/oder AM23, und/oder D25 ist.

14. Pharmazeutische Zusammensetzung, umfassend:
(a) den Antikörper oder den funktionellen Teil davon, oder eine Nukleinsäuresequenz oder ein funktionelles Äquivalent davon, oder einen Vektor, oder eine Zelle, oder eine Kombination nach einem der Ansprüche 1 bis 13 und
(b) ein(en) pharmazeutisch verträglichen/-s Träger, Verdünnungsmittel oder Hilfsstoff.

15. Antikörper oder funktioneller Teil davon, oder eine Nukleinsäuresequenz oder ein funktionelles Äquivalent, oder ein Vektor, oder eine Zelle, oder eine Kombination, oder eine pharmazeutische Zusammensetzung nach einem der Ansprüche 1 bis 14 zur Verwendung als Medikament und/oder prophylaktisches Mittel zwecks zumindest teilweiser Behandlung und/oder Verhütung einer mit RSV im Zusammenhang stehenden Störung.

16. Verfahren zur Produktion eines Antikörpers oder eines funktionellen Teils davon nach Ansprüchen 1 bis 7, umfassend Bereitstellen einer Zelle mit einer Nukleinsäuresequenz oder einem funktionellen Äquivalent davon nach einem der Ansprüche 8 bis 10 oder einem Vektor nach Anspruch 11 und Ermöglichen, dass die Zelle die Nukleinsäuresequenz oder das funktionelle Äquivalent davon oder den Vektor translatiert, wodurch der Antikörper oder der funktionelle Teil davon produziert wird.

17. Verfahren nach Anspruch 16, weiterhin umfassend Ernten, Reinigen und/oder Isolieren des Antikörpers oder des funktionellen Teils davon.

## Revendications

1. Anticorps synthétique ou recombinant, isolé ou une partie fonctionnelle de celui-ci qui est capable de se lier spécifiquement à la protéine F du virus respiratoire syncytial (RSV) et qui comprend:
une séquence de région déterminant la complémentarité (CDR) 1 de chaîne lourde comprenant la séquence KLSIH,
une séquence CDR2 de chaîne lourde comprenant la séquence GYEGEVDEIFYAQKFQH,
une séquence CDR3 de chaîne lourde comprenant la séquence LGVTVTEAGLGIDDY,
une séquence CDR1 de chaîne légère comprenant la séquence RASQIVSRNHLA,
une séquence CDR2 de chaîne légère comprenant la séquence GASSRAT, et
une séquence CDR3 de chaîne légère comprenant la séquence LSSDSSI.

2. Anticorps ou une partie fonctionnelle de celui-ci selon la revendication 1, dans lequel (a) la séquence de chaîne lourde comprend une séquence qui est au moins 90% identique à la séquence:
identisch ist mit der Sequenz 
QVQLVQSGAEVKKPGATVKVSCKISGHTLIKLSIHWVRQAPGKGLEWMGG
YEGEVDEIFYAQKFQHRLTVIADTATDTVYMELGRLTSDDTAVYFCGTLGV
TVTEAGLGIDDYWGQGTLVTVSS (Figure 2);
(b) la séquence de chaîne légère comprend une séquence qui est au moins 90% identique à la séquence: 
EIVLTQSPGTLSLSPGERATLSCRASQIVSRNHLAWYQQKPGQAPRLLIFGA
SSRATGIPVRFSGSGSGTDFTLTINGLAPEDFAVYYCLSSDSSIFTFGPGTKV
DFK (Figure 2);
(c) la séquence de chaîne lourde comprend la séquence: 
QVQLVQSGAEVKKPGATVKVSCKISGHTLIKLSIHWVRQAPGKGLEWMGG
YEGEVDEIFYAQKFQHRLTVIADTATDTVYMELGRLTSDDTAVYFCGTLGV
TVTEAGLGIDDYWGQGTLVTVSS (Figure 2);
(d) la séquence de chaîne légère comprend la séquence: 
EIVLTQSPGTLSLSPGERATLSCRASQIVSRNHLAWYQQKPGQAPRLLIFGA
SSRATGIPVRFSGSGSGTDFTLTINGLAPEDFAVYYCLSSDSSIFTFGPGTKVDFK
(Figure 2);
(e) la séquence de chaîne lourde comprend une séquence qui est au moins 90% identique à la séquence:
QVQLVQSGAEVKKPGATVKVSCKISGHTLIKLSIHWVRQAPGKGLEWMGG YEGEVDEIFYAQKFQHRLTVIADTATDTVYMELGRLTSDDTAVYFCGTLGV TVTEAGLGIDDYWGQGTLVTVSS (Figure ²) et la séquence de chaîne légère comprend une séquence qui est au moins 90% identique à la séquence: 
EIVLTQSPGTLSLSPGERATLSCRASQIVSRNHLAWYQQKPGQAPRLLIFGA
SSRATGIPVRFSGSGSGTDFTLTINGLAPEDFAVYYCLSSDSSIFTFGPGTKVDFK
(Figure 2);
(f) une séquence de chaîne lourde comprend une séquence qui est au moins 90% identique à la séquence: 
QVQLVQSGAEVKKPGATVKVSCKISGHTLIKLSIHWVRQAPGKGLEWMGG
YEGEVDEIFYAQKFQHRLTVIADTATDTVYMELGRLTSDDTAVYFCGTLGV
TVTEAGLGIDDYWGQGTLVTVSS (Figure 2) et la séquence de chaîne légère
 comprend une séquence la séquence: 
EIVLTQSPGTLSLSPGERATLSCRASQIVSRNHLAWYQQKPGQAPRLLIFGA
SSRATGIPVRFSGSGSGTDFTLTINGLAPEDFAVYYCLSSDSSIFTFGPGTKVDFK
(Figure 2);
(g) la séquence de chaîne lourde comprend la séquence: 
QVQLVQSGAEVKKPGATVKVSCKISGHTLIKLSIHWVRQAPGKGLEWMGG
YEGEVDEIFYAQKFQHRLTVIADTATDTVYMELGRLTSDDTAVYFCGTLGV
TVTEAGLGIDDYWGQGTLVTVSS (Figure 2)
 et la séquence de chaîne légère comprend une séquence qui est au moins 90% identique à la séquence: 
EIVLTQSPGTLSLSPGERATLSCRASQIVSRNHLAWYQQKPGQAPRLLIFGA
SSRATGIPVRFSGSGSGTDFTLTINGLAPEDFAVYYCLSSDSSIFTFGPGTKVD
FK (Figure 2); ou
(h) la séquence de chaîne lourde comprend la séquence:
QVQLVQSGAEVKKPGATVKVSCKISGHTLIKLSIHWVRQAPGKGLEWMGG YEGEVDEIFYAQKFQHRLTVIADTATDTVYMELGRLTSDDTAVYFCGTLGV TVTEAGLGIDDYWGQGTLVTVSS (Figure 2) et la séquence de chaîne légère comprend la séquence:

EIVLTQSPGTLSLSPGERATLSCRASQIVSRNHLAWYQQKPGQAPRLLIFGA
SSRATGIPVRFSGSGSGTDFTLTINGLAPEDFAVYYCLSSDSSIFTFGPGTKVDFK
(Figure 2).

3. Anticorps ou une partie fonctionnelle de celui-ci selon la revendication 1 ou 2, qui est conjugué à PEG ou de l'albumine et/ou qui comprend:
- un épitope de liaison de récepteur de sauvetage, et/ou
- des résidus d'acides aminés modifiés identifiés comme étant impliqués dans l'interaction entre Fc et un récepteur FcRN, et/ou
- des résidus d'acides aminés qui ne sont pas d'origine naturelle.

4. Anticorps ou une partie fonctionnelle de celui-ci selon l'une quelconque des revendications 1 à 3, comprenant une région Fc qui comprend une modification à une ou plusieurs positions choisies dans le groupe constitué de 234, 235, 236, 237, 238, 239, 240, 241, 243, 244, 245, 247, 251, 252, 254, 255, 256, 262, 263, 264, 265, 266, 267, 268, 269, 279, 280, 284, 292, 296, 297, 298, 299, 305, 313, 316, 325, 326, 327, 328, 329, 330, 331, 332, 333, 334, 339, 341, 343, 370, 373, 378, 392, 416, 419, 421, 440 et 443 comme il est numéroté par l'index EU tel que présenté dans Kabat et al, ladite modification comprenant une substitution d'acide aminé et/ou une insertion d'acide aminé et/ou une délétion d'acide aminé.

5. Anticorps ou une partie fonctionnelle de celui-ci selon l'une quelconque des revendications 1 à 4, où ledit anticorps ou une partie fonctionnelle de celui-ci possède une constante de dissociation (Kd) de moins de 1 x 10⁻² M, 1 x 10⁻³ M, 1 x 10⁻⁴ M, 1 x 10⁻⁵ M, 1 x 10⁻⁶ M, 1 x 10⁻⁷ M, 1 x 10⁻⁸ M, 1 x 10⁻⁹ M, 1 x 10⁻¹⁰ M, 1 x 10⁻¹¹ M, 1 x 10⁻¹² M, 1 x 10⁻¹³ M, 1 x 10⁻¹⁴ M ou moins de 1 x 10⁻¹⁵ M.

6. Anticorps ou une partie fonctionnelle de celui-ci selon l'une quelconque des revendications 1 à 5 qui est un anticorps humain.

7. Anticorps ou une partie fonctionnelle de celui-ci selon l'une quelconque des revendications 1 à 5, où la partie fonctionnelle de celui-ci est un anticorps à une seule chaîne, un seul fragment variable (sFv), un fragment Fab ou un fragment F(ab')₂.

8. Séquence d'acide nucléique synthétique ou recombinante, isolée, ou un équivalent fonctionnel de celle-ci, codant pour l'anticorps ou une partie fonctionnelle de celui-ci selon l'une quelconque des revendications 1 à 4, 6 ou 7.

9. Séquence d'acide nucléique ou un équivalent fonctionnel de celle-ci selon la revendication 8 qui comprend les séquences:

aaattatccattcac,
ggttatgagggtgaggtcgatgagattttctacgcacagaagttccagcac,
ctaggtgtgacagtgactgaggctggactggggatcgatgactac,
agggccagtcagattgttagcaggaaccacttagcc, ggtgcgtccagtcgggccact und
ctgtcctctgattcctccata

10. Séquence d'acide nucléique ou un équivalent fonctionnel de celle-ci selon la revendication 8 qui comprend la séquence de nucléotides des séquences de chaîne lourde et/ou de chaîne légère dans la Figure 2.

11. Vecteur comprenant une séquence d'acide nucléique ou un équivalent fonctionnel de celle-ci selon l'une quelconque des revendications 8 à 10.

12. Cellule isolée ou recombinante comprenant ou fabriquée pour exprimer la séquence d'acide nucléique ou un équivalent fonctionnel de celle-ci selon l'une quelconque des revendications 8 à 10 et/ou le vecteur selon la revendication 11.

13. Combinaison comprenant:
(a) l'anticorps ou une partie fonctionnelle de celui-ci ou une séquence d'acide nucléique ou un équivalent fonctionnel ou un vecteur ou une cellule selon l'une quelconque des revendications 1 à 12 et
(b) un agent spécifique à RSV différent, de préférence un anticorps, où l'anticorps est de préférence Palivizumab et/ou AM14 et/ou AM16 et/ou AM23 et/ou D25.

14. Composition pharmaceutique comprenant:
(a) l'anticorps ou une partie fonctionnelle de celui-ci ou une séquence d'acide nucléique ou un équivalent fonctionnel de celle-ci ou un vecteur ou une cellule ou une combinaison selon l'une quelconque des revendications 1 à 13 et
(b) un support, un diluant ou un excipient pharmaceutiquement acceptable.

15. Anticorps ou une partie fonctionnelle de celui-ci ou séquence d'acide nucléique ou un équivalent fonctionnel de celle-ci ou vecteur ou cellule ou combinaison ou composition pharmaceutique selon l'une quelconque des revendications 1 à 14 pour une utilisation en tant que médicament et/ou agent prophylactique pour au moins en partie le traitement et/ou la prévention d'un trouble lié à RSV.

16. Méthode pour la production d'un anticorps ou une partie fonctionnelle de celui-ci selon les revendications 1 à 7 consistant à fournir une cellule avec une séquence d'acide nucléique ou un équivalent fonctionnel de celle-ci selon l'une quelconque des revendications 8 à 10 ou un vecteur selon la revendication 11 et à permettre à ladite cellule de traduire ladite séquence d'acide nucléique ou un équivalent fonctionnel de celle-ci ou ledit vecteur, produisant ainsi ledit anticorps ou une partie fonctionnelle de celui-ci.

17. Méthode selon la revendication 16 comprenant en outre la récolte, la purification et/ou l'isolement dudit anticorps ou une partie fonctionnelle de celui-ci.
